# FASCICULE DE BREVET EUROPEEN

(11) **EP 2 958 569 B1**
(45) Date de publication et mention de la délivrance du brevet: **13.01.2021**
(21) Numéro de dépôt: 14711831.9
(22) Date de dépôt: 25.02.2014
(51) Int. Cl.: A61K 31/665, A61P 35/00, A61K 45/06

(54) **HÉTÉROCYCLES PHOSPHORÉS ANALOGUES DE SUCRES À ACTIVITÉ ANTIMÉTASTATIQUE**
ZUCKERANALOGE PHOSPHORHALTIGE HETEROCYCLEN MIT ANTIMETASTATISCHER WIRKUNG
SUGAR-ANALOG PHOSPHORUS-CONTAINING HETEROCYCLES HAVING AN ANTI-METASTATIC ACTIVITY

(30) Priorité: 25.02.2013 FR 1351654
(43) Date de publication de la demande: 30.12.2015
(73) Titulaire: Centre National de la Recherche Scientifique (C.N.R.S.), 75016 Paris (FR); Ecole Nationale Superieure De Chimie Montpellier (ENSC), 34000 Montpellier (FR); Université de Montpellier, 34090 Montpellier (FR); UNIVERSITE PARIS-NORD 13, 93430 Villetaneuse (FR); Université Paris-Sud 11, 91400 Orsay (FR)
(72) Inventeur: BAKALARA, Norbert, F-34380 Saint Martin De Londres (FR); DELAFORGE, Marcel, 77122 Mothyon (FR); LECOUVEY, Marc, F-75016 Paris (FR); HUGNOT, Jean-Philippe, F-34000 Montpellier (FR); LEGRAND, Philippe, F-34170 Castelnau Le Nez (FR); PIRAT, Jean-Luc, F-34130 Saint-Aunes (FR); VIRIEUX, David, F-34000 Montpellier (FR); VOLLE, Jean-Noël, F-34830 Jacou (FR)
(74) Mandataire: Jacobacci Coralis Harle
(86) Numéro de dépôt international: PCT/FR2014/050409
(87) Numéro de publication internationale: WO 2014/128429

(56) Documents cités:
- WO-A1-2009/004096
- WO-A1-2013/025418
- Ludovic Clarion ET AL: "Oxaphosphinanes: New Therapeutic Perspectives for Glioblastoma", J Med Chem, 23 janvier 2012 (2012-01-23), pages 2196-2211, XP055073745, DOI: 10.1021/jm201428a| Extrait de l'Internet: URL:http://pubs.acs.org/doi/pdf/10.1021/jm 201428a [extrait le 2013-07-31] cité dans la demande
- SORENSEN M D ET AL: "Cyclic phosphinamides and phosphonamides, novel series of potent matrix metalloproteinase inhibitors with antitumour activity", BIOORGANIC & MEDICINAL CHEMISTRY, PERGAMON, GB, vol. 11, no. 24, 1 janvier 2003 (2003-01-01), pages 5461-5484, XP002503671, ISSN: 0968-0896, DOI: 10.1016/J.BMC.2003.09.015
- LISA VOLK-DRAPER ET AL.: "Paclitaxel therapy promotes breast cancer metastasis in a TLR4-dependent manner", CANCER RES, vol. 74, no. 19, 1 October 2014 (2014-10-01), pages 5421-5434,
- YI SEOK CHANG ET AL.: "Stress-inducible gene Atf3 in the noncancer host cells contributes to chemotherapy-exacerbated breast cancer metastasis", PROC NATL ACAD SCI U S A., 7 August 2017 (2017-08-07), pages e7159-e7168,

## Description

### DOMAINE DE L'INVENTION

La présente invention se rapporte au domaine du traitement thérapeutique des cancers chez l'homme ou l'animal. L'invention concerne en particulier l'utilisation en médecine humaine ou vétérinaire d'une famille d'analogues phosphorés de saccharides (glycomimétiques), incorporant un cycle phosphinane, de préférence oxaphosphinane, pour son activité antimétastatique.

### ART ANTERIEUR

Selon des statistiques récentes, le cancer représente la deuxième cause de mortalité après les maladies cardiovasculaires dans les pays industrialisés. Les moyens mis en œuvre pour faire face aux maladies cancéreuses passent par le renforcement du diagnostic précoce, mais aussi par l'amélioration des traitements médicamenteux. La découverte de nouvelles molécules originales, dont la spécificité à l'égard des cellules tumorales serait totale par rapport aux cellules saines, permettrait la mise au point de nouvelles thérapies.

Avant d'effectuer un choix thérapeutique pour le traitement d'un cancer, un certain nombre de paramètres doivent être étudiés : le type de cancer (sarcomes, mélanomes...), l'organe atteint, le stade d'évolution du cancer, l'ensemble des facteurs pronostics et les caractéristiques propres au malade (âge, état général, état psychique...). A partir de ces données, une thérapie peut être choisie, soit locale, soit générale. Les traitements les plus efficaces sont des thérapies locales mettant en jeu des méthodes de chirurgie et/ou de radiothérapie. Elles traitent les lésions peu développées et guérissent la plupart des cancers localisés. Les thérapies générales, chimiothérapie et/ou l'hormonothérapie, sont en général des traitements palliatifs ou adjuvants. Ces traitements sont mis en œuvre dans le cas de cancers localisés mais plus développés. Ils permettent de guérir un nombre limité de cancers généralisés mais améliorent l'espérance de vie des malades (cf. Capdeville R., Buchdunger E., Zimmermann J., Matter A., Nature Rev. Drug Discov., 2002, 1, 493; Eisenberg B. L., Von Mehren M., Expert Opin Pharmacother., 2003, 6, 869; Gilman A., Philips F. S., Science, 1946, 103, 409; Gingras D, Béliveau R. Med. Sci., 1997, 13, 1428-35.

Le phénomène de métastase est une série complexe d'étapes au cours desquelles des cellules cancéreuses quittent le site originel (tumeur primaire) et migrent vers d'autres parties du corps. Des cellules cancéreuses peuvent en effet se détacher d'une tumeur primaire, et, si le système immunitaire ne les détecte pas, pénétrer dans les vaisseaux lymphatiques et sanguins puis circuler vers une autre partie du corps pour y former une nouvelle tumeur (*« métastaser »*). On parle de tumeur secondaire ou métastatique.

La formation de métastases est affectée par de nombreux facteurs, et dépend notamment du type de cancer et de son agressivité. Le risque de métastase s'accroît avec la durée de la présence de la tumeur primaire dans le corps, et c'est la capacité de s'étendre à d'autres tissus et d'autres organes qui fait du cancer une maladie potentiellement mortelle. La plupart des personnes qui décèdent d'un cancer présentent des métastases au moment de leur décès, la dissémination métastatique étant la cause la plus fréquente de décès des patients cancéreux.

Les cancers les plus fréquents (prostate, sein, côlon, poumon, par exemple) se développent dans les organes qui peuvent être totalement ou partiellement enlevés par chirurgie. La chirurgie peut guérir les patients si le cancer primaire n'a pas métastasé. La plupart des conséquences graves de ces cancers survient en raison de leur propagation à d'autres parties du corps. Dans certains cas, l'effet le plus grave de cancer est sa propagation à une partie particulièrement essentielle du corps. Dans d'autres cas, la propagation et la croissance dans de nombreux organes crée tant de cellules cancéreuses que le métabolisme normal de l'organisme en est profondément perturbé.

La découverte d'une tumeur primitive conduit généralement le médecin à rechercher des foyers secondaires. Malgré la précision des moyens disponibles, le bilan établi peut rester négatif alors que des mois ou des années plus tard, l'apparition de métastases visibles indique qu'elles étaient présentes mais minimes et « occultes » précédemment. Un cancer n'est généralement détectable qu'à partir du moment où il atteint un certain volume : il est rare que l'on puisse le détecter avant qu'il atteigne 1 cm de diamètre, ce qui correspond à environ 1 milliard de cellules.

Le traitement d'un cancer ne se résume donc pas au traitement de la tumeur primaire (traitement antiprolifératif local), mais doit bien souvent s'accompagner d'un traitement antimétastatique préventif, en particulier lorsque le risque que le cancer métastase est élevé, afin d'espérer une rémission complète et prolongée. En effet, lors des traitements par chimiothérapie l'action du principe actif antiprolifératif fait émerger des cellules à fort potentiel migratoire. Cette réponse cellulaire au traitement concerne une sous-population cellulaire (de type cellule souche) qui possède une plasticité moléculaire lui autorisant une réponse adaptée aux variations environnementales. Le traitement antimétastatique a pour but de bloquer cette réponse adaptative d'échappement au traitement au travers de la migration.

Dans certains cas, la tumeur primitive n'est pas décelée, soit que celle-ci est restée très petite et invisible, soit qu'elle a connu une régression spontanée après avoir libéré les cellules malignes responsables des foyers métastatiques.

La limitation concernant l'utilisation des anticancéreux connus concerne leur forte toxicité, qui est à l'origine d'un grand nombre d'effets secondaires pouvant aller jusqu'à la mort du patient. Les armes chimiques utilisées pour le traitement du cancer sont censées détruire les cellules cancéreuses en épargnant les cellules saines. Mais la sélectivité est toute relative, et la plupart des médicaments utilisés en chimiothérapie présentent une toxicité hématologique non négligeable. Réduire les effets secondaires néfastes, surtout ceux aux conséquences graves sur le plan médical et psychologique, est aussi important que de tenter d'améliorer l'efficacité d'un médicament donné. Dans l'ensemble, l'arsenal actuel du chimiothérapeute se compose encore de médicaments forts cytotoxiques, anciens, la plupart des anticancéreux connus ayant déjà plusieurs dizaines d'années, bien peu ciblés, sur le plan cellulaire tout au moins, et n'offrant aucune alternative aux phénomènes de résistance. Il existe donc un besoin en nouvelles molécules anticancéreuses, utilisables en chimiothérapie, qui, idéalement, ne cibleraient que les cellules cancéreuses, et présenteraient des propriétés antimétastatiques.

Les sucres, qui représentent une famille de biomolécules omniprésentes dans le monde du vivant, avec une grande variété de structures et de fonctions, présentent de nombreuses applications en thérapie : la lutte contre l'obésité, le diabète, mais aussi en tant qu'antivirus, antibiotiques et anticancéreux. La synthèse d'analogues de sucres ou glycomimétiques est intéressante, dans la mesure où ces composés sont susceptibles d'interférer avec les différents récepteurs ou enzymes faisant intervenir des sucres, en particulier, les mécanismes énergétiques ou de biosynthèse de certaines molécules, de glycoconjugués, et les mécanismes d'adhérence entre cellules.

Dans le domaine de la préparation d'analogues saccharidiques cycliques, deux grands axes ont déjà été développés : le remplacement du groupe hydroxyle en position anomérique par un groupement carboné (C-arylglycosides), et le remplacement de l'atome d'oxygène intracyclique par un autre hétéroatome (phosphosucres, phosphasucres, iminosucres, thioglycosides).

Récemment, il a été montré dans la demande WO 2009/004096 et l'article Clarion L. et al., J Med. Chem. 2012, 2196-2211, qu'une famille de phosphinosucres présentait des propriétés anticancéreuses. La structure de quelques composés représentatifs de cette famille est indiquée sur le schéma ci-dessous :

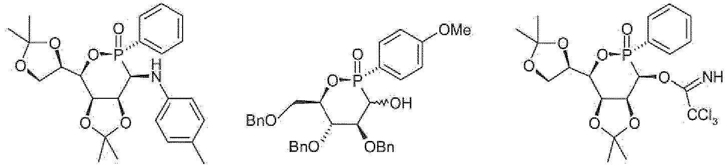

Les composés de cette famille présentent une activité antiproliférative (cytotoxicité à faible dose) vis-à-vis de plusieurs lignées cellulaires cancéreuses, sans présenter de cytotoxicité vis-à-vis de cellules saines (non tumorales) aux doses pour lesquelles ils possèdent une activité anticancéreuse.

De façon surprenante, les présents inventeurs ont maintenant découvert que ces composés phosphiniques hétérocycliques, analogues de sucres, présentaient en outre des propriétés antimétastatiques. Par propriétés antimétastatiques, on entend la propriété de réduire ou prévenir l'apparition ou la propagation de métastases chez un patient atteint d'un cancer. Ceci peut se traduire par une élimination de la formation de métastases ou par une réduction de leur taille et/ou du nombre de sites de métastases par rapport à l'état qui serait constaté en l'absence de traitement.

Au sens de l'invention, le traitement antiprolifératif d'une tumeur cancéreuse secondaire ou métastatique (par exemple faire régresser des métastases établies ou inhiber la transformation de métastases occultes en macrométastases) n'est pas considéré comme un traitement antimétastatique mettant en jeu les propriétés antimétastatiques des composés selon l'invention.

Les composés selon l'invention ciblent donc non seulement la multiplication des cellules cancéreuses en altérant la croissance de la tumeur primaire ou de tumeurs métastatiques (action curative antiproliférative), mais ciblent également leur mobilité et inhibent ou du moins réduisent ainsi la formation de métastases (action préventive).

Sans vouloir être liés par une théorie, les inventeurs pensent que les composés selon l'invention inhibent ou réduisent le processus moléculaire de développement de métastases et préviennent donc ou réduisent l'essaimage des métastases à partir de tumeurs primaires.

### RESUME DE L'INVENTION

L'invention a pour objet des composés de formule (1) suivante, dénommés phosphinosucres dans le cadre de la présente invention, pour réduire ou prévenir l'apparition de métastases chez un patient atteint d'un cancer : 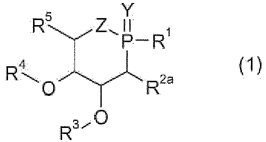
Dans cette formule, Y représente un atome d'oxygène, de soufre ou de sélénium, de préférence un atome d'oxygène,
Z représente O, S, Se, NH ou un groupe NR⁶, dans lequel R⁶ est un groupe aryle ou alkyle éventuellement substitué, de préférence un atome d'oxygène,
R¹ représente un atome d'hydrogène, un groupe alkyle éventuellement substitué, ou un groupe aryle,
R^{2a} représente un atome d'hydrogène, d'halogène, notamment un atome de fluor, un groupe azoture (N₃), un groupe carbonate ou dithiocarbonate, un groupe 1H-[1,2,3]-triazolyle ou un groupe -X-R², X représentant un atome d'oxygène, de soufre, de sélénium, un groupe NH ou NR⁷, dans lequel R⁷ est un groupe aryle ou alkyle éventuellement substitué, X représentant de préférence O ou NH, et R² représentant un groupe aryle, alkyle éventuellement substitué, un atome d'hydrogène, un groupe trichloroacétimidate (-C(=NH)CCl₃), acyle, formyle, sulfonyle, sulfinyle, *tert*-butyldiphénylsilyle, allyle, un groupe saccharyle, ester, amide, thioamide, sulfonamide, ou bien X-R² représente un groupe P(O)R^{2'}R^{6'} dans lequel R^{2'} et R^{6'} désignent, indépendamment l'un de l'autre, un groupe aryle, alkyle éventuellement substitué, OH, alcoxy ou aryloxy,
R³ et R⁴ représentent, indépendamment l'un de l'autre, un groupe aryle, alkyle éventuellement substitué, un atome d'hydrogène, un groupe trichloroacétimidate, acyle, formyle, sulfonyle, sulfinyle, *tert*-butyldiphénylsilyle, allyle, ester, amide, thioamide, sulfonamide, saccharyle, le groupe saccharyle dérivant de sucres ou de leurs analogues substitués ou déoxy, ou bien R³ et R⁴, pris conjointement, forment un radical bivalent de formule -R³-R⁴-, dans laquelle -R³-R⁴- représente de préférence un groupe isopropylydène, benzylidène, diphényl méthylidène, cyclohexyl méthylidène, et leurs analogues substitués, par exemple un groupe 4-méthoxybenzylidène, ou un groupe alkylène linéaire tel que le groupe éthylène (de façon à former un groupe propane-1,2-diol),
R⁵ représente un atome d'hydrogène ou un groupe hydrocarboné comprenant un ou plusieurs hétéroatomes choisis de préférence parmi l'oxygène, le soufre ou l'azote, mieux l'oxygène,
   L'invention fournit aussi des compositions pharmaceutiques anticancéreuses pour une utilisation en médecine humaine ou vétérinaire, qui comprennent au moins un composé de formule (1) en association avec un ou plusieurs excipients et/ou véhicules pharmaceutiquement acceptables, pour réduire ou prévenir l'apparition de métastases chez un patient atteint d'un cancer.

L'invention concerne aussi une méthode de traitement thérapeutique d'un homme ou d'un animal, pour réduire ou prévenir l'apparition de métastases chez un sujet nécessitant un tel traitement, ladite méthode comprenant une étape au cours de laquelle on administre à l'homme ou l'animal une dose thérapeutiquement efficace d'un composé de formule (1) tel que défini dans la présente description, soit seul, soit en mélange avec un ou plusieurs excipients et/ou véhicules pharmaceutiquement acceptables.

### DESCRIPTION DES FIGURES

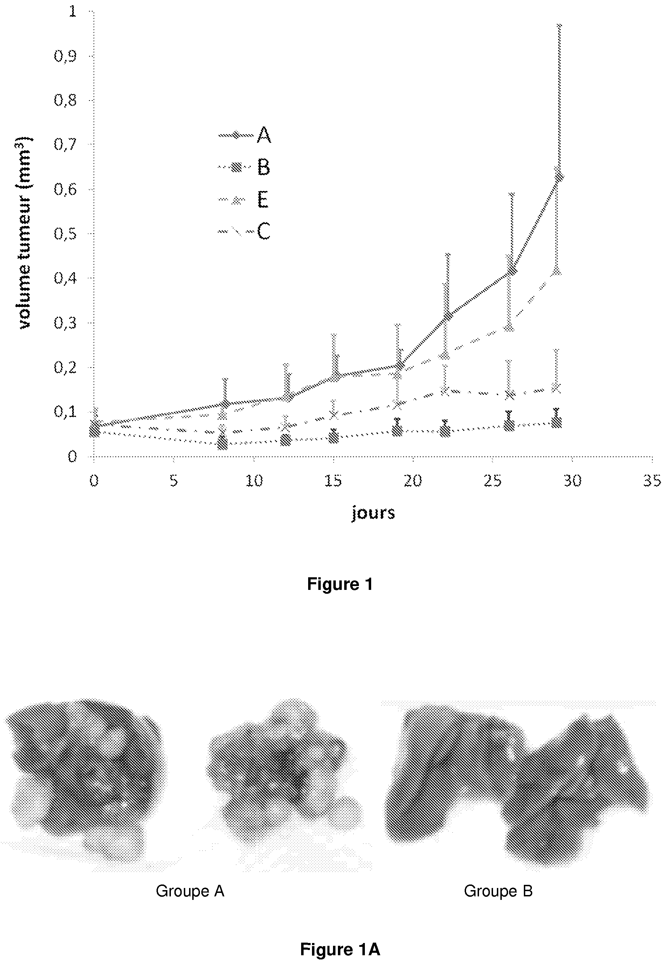

La figure 1 présente une mesure de la taille des tumeurs présentées par des souris ayant subi une implantation sous cutanée de cellules cancéreuses de la lignée SNB75 (5x10⁶ cellules) en fonction du temps et de différents traitements. L'écart type moyen est également représenté pour chaque mesure.

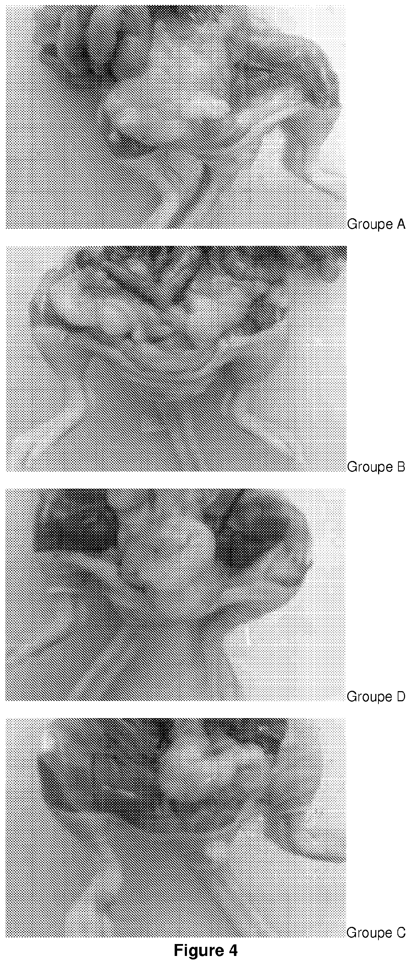

La figure 1A et la figure 4 présentent des photographies d'organes de souris (foie ou ovaires) ayant développé ou non des métastases suivant qu'elles ont reçu ou non un traitement par un composé selon l'invention (**3.48a** ou **4.2a**), après qu'elles aient reçu une injection de cellules cancéreuses.

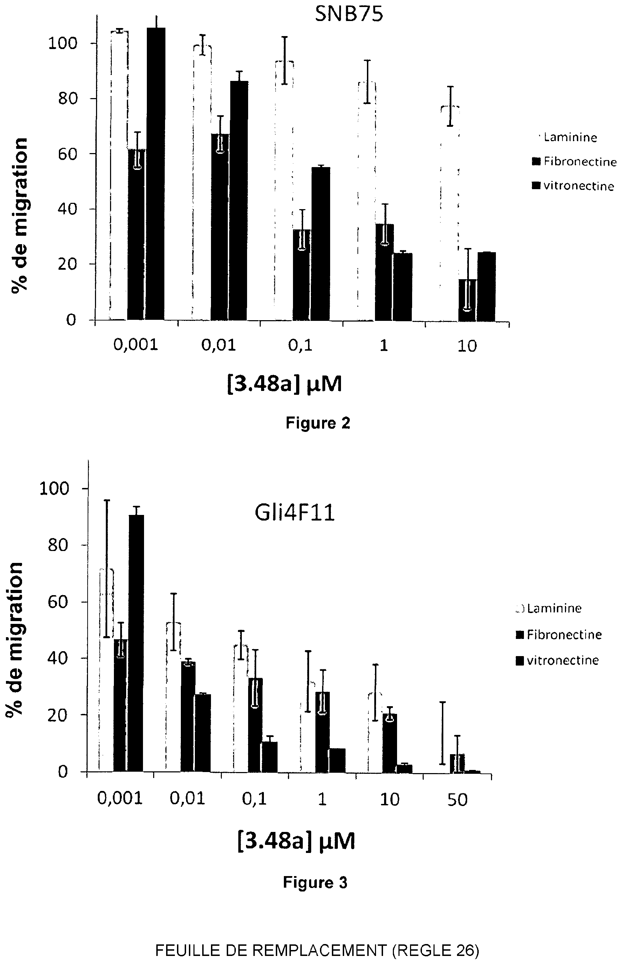

Les figures 2 et 3 représentent des diagrammes illustrant la migration de cellules cancéreuses des lignées SNB75 et Gli4F11, en chambre de Boyden, sur différents supports de protéines de la matrice extracellulaire, en fonction de la concentration en composé selon l'invention **3.48a.**

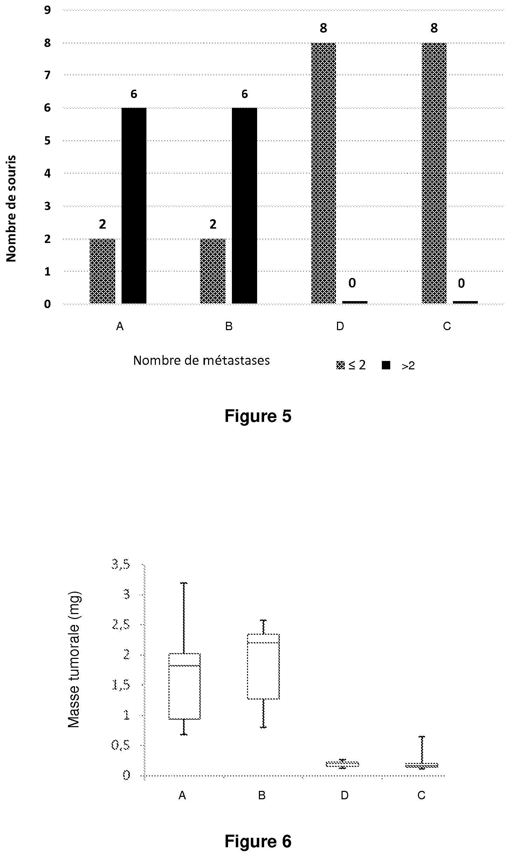

La figure 5 présente le nombre moyen de métastases développées par des souris après qu'elles aient reçu une injection de cellules cancéreuses, selon qu'elles ont été traitées ou non par le composé selon l'invention **4.2a.** La figure 6 représente la répartition de la masse de ces tumeurs secondaires en fonction des conditions de traitement.

### DESCRIPTION DETAILLEE DE L'INVENTION

Il est fourni selon l'invention une famille de composés phosphinosucres comprenant un cycle à 6 chaînons incorporant un atome de phosphore (cycle phosphinane), typiquement une famille de 1,2-oxaphosphinanes-2-oxyde, en particulier pour son activité antimétastatique.

Dans la description des composés chimiques, les termes sont généralement employés selon leur signification habituelle.

Le terme "alkyle" ou "cycloalkyle" signifie dans la présente demande selon le cas un radical hydrocarboné linéaire ou ramifié, saturé ou insaturé, ayant de 1 à 25 atomes de carbone, incluant notamment les groupes acycliques ayant de 1 à 8 atomes de carbone tels que les groupes méthyle, éthyle, n-propyle, isopropyle, butyle, n-hexyle, les groupes cycloalkyle ayant de préférence de 3 à 7 atomes de carbone, les groupes cycloalkylméthyle ayant de préférence de 4 à 8 atomes de carbone.

Dans le présent texte, une description relative à un groupe alkyle est également applicable à un groupe cycloalkyle.

Par groupe "alkyle substitué," on entend un groupe alkyle tel que défini ci-dessus, connecté par l'intermédiaire d'un atome de carbone sp³ et substitué par un ou plusieurs groupes aryle et/ou comprenant un ou plusieurs hétéroatomes tels que N, S ou O. On citera à titre d'exemples les groupes arylalkyle tels que le groupe trityle (-CPh₃), le groupe benzyle noté Bn ou le groupe 4-méthoxybenzyle, les groupes alcoxyalkyle, notamment les groupes dialcoxyméthyle tels que les groupes diéthoxyméthyle ou diméthoxyméthyle, les groupes CH₂CO₂R¹¹, dans lesquels R¹¹ représente un groupe alkyle éventuellement substitué ou aryle.

Le terme "alcoxy" désigne un groupe alkyle connecté au reste de la molécule par un atome d'oxygène, par exemple un groupe éthoxy, méthoxy, ou n-propoxy.

Le terme "aryloxy" désigne un groupe aryle connecté au reste de la molécule par un atome d'oxygène, par exemple un groupe benzoxy.

Le terme "acyle" désigne un radical dérivant d'un acide carboxylique par la suppression du groupe hydroxyle, ayant de préférence pour formule -C(O)R⁸, R⁸ désignant un groupe aryle ou alkyle éventuellement substitué, par exemple les groupes acétyle, propionyle, oléoyle, myristoyle, benzoyle, trifluoro-acétyle.

Le terme "sulfonyle" désigne un radical dérivant d'un acide sulfonique par la suppression du groupe hydroxyle, ayant de préférence pour formule -SO₂R⁹, R⁹ désignant un groupe aryle ou alkyle éventuellement substitué, par exemple un groupe CF₃.

Le terme "sulfinyle" désigne un radical dérivant d'un acide sulfinique par la suppression du groupe hydroxyle, ayant de préférence pour formule -SOR¹⁰, R¹⁰ désignant un groupe aryle ou alkyle éventuellement substitué.

Le terme "dithiocarbonate" désigne un groupe de formule -OC(S)SR^{9c}, R^{9c} désignant un groupe aryle ou alkyle éventuellement substitué.

Le terme "carbonate" désigne un groupe de formule -OC(O)OR^{9d}, R^{9d} désignant un groupe aryle ou alkyle éventuellement substitué.

Le terme "groupe ester" désigne un groupe de formule -C(O)OR^{10'}, R^{10'} désignant un groupe aryle ou alkyle éventuellement substitué.

Le terme "groupe amide" désigne un groupe de formule -C(O)NR^{9'}R^{9"}, R^{9'} désignant un groupe aryle ou alkyle éventuellement substitué et R^{9"} désignant un groupe aryle ou alkyle éventuellement substitué ou un atome d'hydrogène, par exemple un groupe -C(O)NHPh.

Le terme "groupe thioamide" désigne un groupe de formule -C(S)NR^{9a}R^{9b}, R^{9a} désignant un groupe aryle ou alkyle éventuellement substitué et R^{9b} désignant un groupe aryle ou alkyle éventuellement substitué ou un atome d'hydrogène.

Le terme "groupe sulfonamide" désigne un groupe de formule -SO₂NR^{11'}R^{11"}, R^{11'} désignant un groupe aryle ou alkyle éventuellement substitué et R^{11"} désignant un groupe aryle ou alkyle éventuellement substitué ou un atome d'hydrogène.

Le terme "aryle" désigne un radical carbocyclique monovalent aromatique comportant un seul cycle (par exemple un groupe phényle) ou de multiples cycles condensés (par exemple les groupes naphthyle, terphényle). Le groupe aryle ou le groupe hétéroaryle défini ci-dessous peut éventuellement être substitué par un ou plusieurs groupes tels que, sans limitation, les groupes alkyle (par exemple méthyle), hydroxyalkyle, amino-alkyle, hydroxyle, thiol, amino, halogéno (fluoro, bromo, iodo, chloro), nitro, alkylthio, alcoxy (par exemple méthoxy), aryloxy, mono-alkylamino, dialkylamino, acyle, carboxyle, alcoxycarbonyle, aryloxycarbonyle, hydroxysulfonyle, alcoxysulfonyle, aryloxysulfonyle, alkylsulfonyle, alkylsulfinyle, cyano, trifluorométhyle, tetrazolyle, carbamoyle, alkylcarbamoyle, dialkylcarbamoyle. Alternativement, deux positions adjacentes du cycle aromatique peuvent être substituées par un groupe méthylènedioxy ou éthylènedioxy.

Le terme "heteroaryle" désigne un radical carbocyclique monovalent aromatique comportant un seul cycle (par exemple un groupe phényle) ou de multiples cycles condensés (par exemple les groupes naphthyle, terphényle) où un ou plusieurs atomes de carbone du ou des cycles aromatiques sont substitués par un hétéroatome tel que l'azote, l'oxygène, le phosphore ou le soufre. Les groupes hétéroaryle peuvent être des structures à un seul ou plusieurs cycles aromatiques, ou des structures à un seul ou plusieurs cycles aromatiques couplés avec un ou plusieurs cycles non aromatiques. Dans les structures possédant plusieurs cycles, les cycles peuvent être fusionnés, liés de façon covalente ou liés ensemble par l'intermédiaire d'un groupe commun divalent tel qu'un groupe méthylène, éthylène, carbonyle. Des exemples de groupes hétéroaryle sont les groupes thiophène (2-thiényl, 3-thiényl), pyridine (2-pyridyl, 3-pyridyl, 4-pyridyl), isoxazole, phthalimide, pyrazole, indole, furane et leurs analogues benzofusionnés, phényl pyridyl cétone, quinoline, phénothiazine, carbazole, benzopyranone.

Dans le présent texte, une description relative à un groupe aryle est également applicable à un groupe hétéroaryle.

Le terme "groupe saccharyle," tel qu'employé ici, englobe tous les radicaux dérivant par la suppression d'un groupe hydroxyle ou d'un atome d'hydrogène (de préférence un groupe hydroxyle) d'un carbohydrate ou sucre, naturel ou synthétique, protégé ou non protégé. Le groupe saccharyle inclut les groupes monosaccharyle et oligosaccharyle tels que les groupes disaccharyle. Les groupes saccharyle, par exemple les groupes glucosyle, mannosyle, peuvent dériver de sucres tels que, sans limitation, l'acide glucuronique, le lactose, saccharose, maltose, allose, alltrose, glucose, mannose, idose, galactose, talose, ribose, arabinose, xylose, lyxose, fructose, thréose, érythrose, β-D-N-acétylgalactosamine, β-D-N-acétylgalactosamine, fucose, acide sialique, acide N-acétylneuraminique, acide N-acétylmuramique, glucosamine, galactosamine, rhamnose et leurs analogues protégés ou substitués, par exemple par des groupes acyle, alkyle, aryle, halogéno, amino, ainsi que leurs analogues déoxy. Par groupe oligosaccharyle, on entend un groupe saccharyle dérivant d'au moins deux monosaccharides liés de façon covalente, comprenant de préférence de 1 à 3 unités saccharide. Les groupes saccharyle préférés sont les groupes monosaccharyle. Dans les composés de formule (1), lorsque R^{2a} représente -X-R², R² étant un groupe saccharyle, ce groupe saccharyle est de préférence lié par un groupe X représentant O ou NH, de préférence O. Pour une description de structures de type saccharide, on pourra se référer à l'ouvrage "Essentials of Glycobiology," Varki et al. Eds., chapitre 2 (Cold Spring Harbor Press, Cold Spring Harbor, N.Y., 1999).

Par saccharide, on entend un monosaccharide ou un oligosaccharide. Bn représente le groupe benzyle, Ac le groupe acétyle.

Certains composés de l'invention peuvent exister aussi bien sous forme solvatée que non solvatée, par exemple sous forme hydratée. En général, les formes solvatées sont équivalentes aux formes non solvatées et sont comprises dans la portée de la présente invention. Certains composés de la présente invention peuvent exister sous de multiples formes cristallines ou amorphes. En général, toutes les formes physiques sont équivalentes pour les utilisations envisagées par la présente invention et sont comprises dans la portée de la présente invention.

Les composés selon l'invention possèdent plusieurs centres asymétriques (optiques), de sorte que des énantiomères ou des diastéroisomères peuvent exister. Il est entendu que l'invention s'étend à tous les énantiomères et diastéroisomères des composés de formule (1) et à leurs mélanges, notamment les racémates. En d'autres termes, les composés selon l'invention peuvent être utilisés sous la forme d'un énantiomère purifié ou sous la forme d'un mélange d'énantiomères. Les différents isomères peuvent être séparés selon des méthodes connues de l'homme du métier, notamment par chromatographie sur gel de silice ou par cristallisation fractionnée.

Les composés de formule (1) préférés sont ceux dans lesquels Y = Z = O, c'est-à-dire des 1,2-oxaphosphinanes-2-oxydes.

Dans les composés selon l'invention, le substituant R¹, lorsqu'il ne désigne pas un atome d'hydrogène, est toujours connecté à l'atome de phosphore intracyclique par l'intermédiaire d'un atome de carbone.

Les groupes R¹ préférés sont les groupes H, alkyle, tel que le groupe 2-benzyloxyéthyle, éthyle, n-butyle, 3-phénylpropyle, n-octyle, dialcoxyméthyle tels que les groupes diéthoxyméthyle ou diméthoxyméthyle, aryle, tel que les groupes phényle, 4-méthylphényle, 4-nitrophényle, 4-aminophényle, 4-méthoxyphényle, 3,4-difluorophényle, 3,5-difluorophényle, 2-thiényle, 4-fluorophénylé, 4-biphénylé, 3-méthylphényle, 3-méthoxyphénylé ainsi que les groupes suivants :

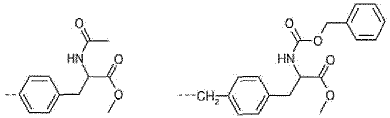

Les groupes R² préférés sont les groupes H, arylsulfonyle, méthylsulfonyle, trichloroacétimidate, benzyle, saccharyle, et aryle, tel que phényle, 4-méthylphényle, 4-nitrophényle, 4-aminophényle, 3,4-difluorophényle, 3,5-difluorophényle, 3,4-dinitrophényle.

Les groupes X-R² préférés sont les groupes O-aryle, OH, NH₂, NH-aryle, S-aryle, dithiocarbonate, NHCH₂CO₂R¹¹, R¹¹ ayant la signification indiquée ci-dessus, NHC(O)R¹², R¹² représentant un groupe alkyle éventuellement substitué ou aryle, O-SO₂R⁹, R⁹ ayant la signification indiquée ci-dessus, NH-Bn, O-saccharyle, OC(=NH)CCl₃, acide phosphonique, acide phosphinique ou oxyde de phosphine, urée, thiourée, carbamate, carbonate.

De préférence, R³ et R⁴ représentent, indépendamment l'un de l'autre, un atome d'hydrogène, un groupe benzyle, benzoyle, acétyle, ou forment conjointement un radical bivalent de formule -R³-R⁴- représentant de préférence le groupe isopropylydène.

Selon un mode de réalisation préféré de l'invention, le groupe R⁵ est tel que les composés (1) répondent aux formules (2) ou (3) : 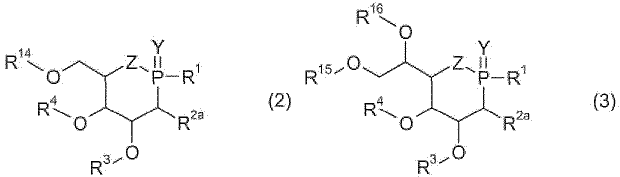 dans lesquelles R¹, R^{2a}, R³, R⁴, Y et Z ont les mêmes significations que précédemment, R¹⁴, R¹⁵ et R¹⁶ représentent, indépendamment les uns des autres, un atome d'hydrogène, un groupe aryle, alkyle éventuellement substitué, un groupe trichloroacétimidate, acyle, formyle, sulfonyle, sulfinyle, *tert*-butyldiphénylsilyle, allyle, ester, amide, thioamide, sulfonamide, saccharyle ou bien R¹⁵ et R¹⁶, pris conjointement, forment un radical bivalent de formule -R¹⁵-R¹⁶-, dans laquelle -R¹⁵-R¹⁶- représente de préférence un groupe isopropylydène, benzylidène, diphényl méthylidène, cyclohexyl méthylidène, et leurs analogues substitués, par exemple un groupe 4-méthoxybenzylidène, ou un groupe alkylène linéaire tel que le groupe éthylène.

Le groupe R⁵, lorsqu'il ne représente pas un atome d'hydrogène, comprend de préférence de 1 à 25 atomes de carbone, de préférence de 1 à 20 atomes de carbone, mieux de 1 à 10 atomes de carbone, et encore mieux de 1 à 8 atomes de carbone. Le groupe R⁵ peut être un groupe alkyle éventuellement substitué comprenant un ou plusieurs hétéroatomes choisis de préférence parmi l'oxygène, le soufre ou l'azote, mieux l'oxygène. Les groupes R⁵ préférés sont les groupes alcoxyalkyle tels que le groupe benzyloxyméthyle (-CH₂OBn), - CH₂OH, 2,2-diméthyl-[1,3]-dioxolan-4-yle et 1,2-dihydroxy-éthyle CH(OH)CH₂OH, ce qui se traduit, dans les formules (2) et (3) par R¹⁴ = H ou Bn, et R¹⁵ = R¹⁶ = H ou R¹⁵ et R¹⁶, pris conjointement, forment un radical isopropylydène.

Les différentes méthodes de préparation des composés de formule (1) ont été décrites de façon détaillée dans la demande WO 2009/004096, et l'article Clarion L. et al. J Med. Chem. 2012, 2196-2211.

Des exemples de familles préférées de composé (1) sont celles ayant les formules générales suivantes, où R¹, R^{2a} sont tels que définis précédemment :

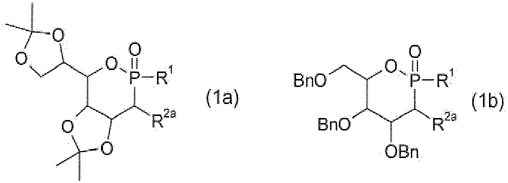

Parmi les 1,2-oxaphosphinanes de formule générale (18), les composés de formules (19) et (20) sont préférés, où R¹ et R³ à R⁵ sont tels que définis précédemment, R¹ représentant de préférence un groupe aryle, un atome d'hydrogène, un groupe alkyle optionnellement substitué tel qu'un un groupe dialcoxyméthyle :

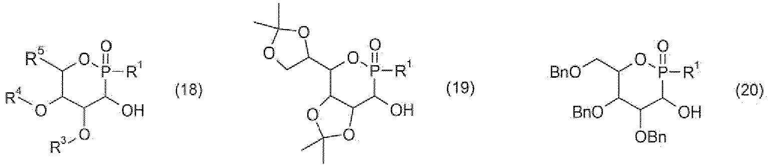

Une seconde classe préférée de composés (1) selon l'invention correspond aux 1,2-oxaphosphinanes de formule générale (21) : 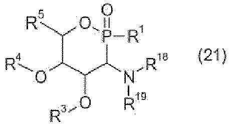 où R¹, R³ à R⁵ ont la même signification que précédemment, R¹⁸ et R¹⁹ représentent indépendamment l'un de l'autre un atome d'hydrogène, un groupe aryle, un groupe alkyle éventuellement substitué, un groupe trichloroacétimidate, acyle, formyle, sulfonyle, sulfinyle, *tert*-butyldiphénylsilyle, allyle, ester, amide, thioamide, sulfonamide, R¹⁸ représentant de préférence un atome d'hydrogène. Parmi ces phosphinosucres azotés, on peut citer ceux comportant un groupe amino en position C₃(R¹⁸ = R¹⁹ = H), un groupe benzylamino en position C₃(R¹⁸ = H, R¹⁹ = Bn) ou un groupe acétylamino en position C₃(R¹⁸ = H, R¹⁹ = Ac). Parmi les composés (21), les composés de formule (22) et (23) sont préférés, R¹ et R¹⁹ ayant la signification indiquée précédemment, R¹ représentant de préférence un groupe aryle, un atome d'hydrogène ou un groupe dialcoxyméthyle et R¹⁹ représentant de préférence un groupe aryle ou acyle :

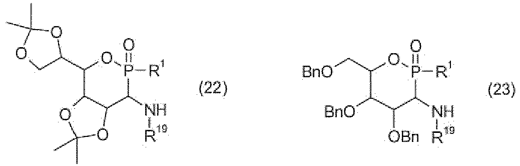

Une troisième classe de composés (1) préférée correspond aux composés suivants de formule (24), dans laquelle les différents substituants ont les significations indiquées précédemment et R²⁰ désigne un groupe saccharyle, de préférence monosaccharyle.

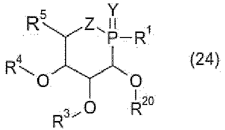

Parmi les composés de formule (24), les composés préférés sont les pseudo-disaccharides de formules (27), (27a) et 27b) suivants (issus d'un couplage avec un dérivé de mannose ou de glucose), où R^{19a}, R1^{9b}, R^{19c}, R^{19d} R^{1ge}, R^{19f}, et R^{19g} désignent indépendamment les uns des autres un atome d'hydrogène ou le groupe benzyle, R^{19h} désigne un atome d'hydrogène ou le groupe méthyle, les autres substituants étant tels que définis précédemment :

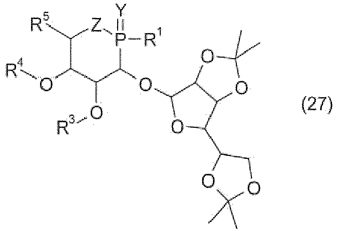

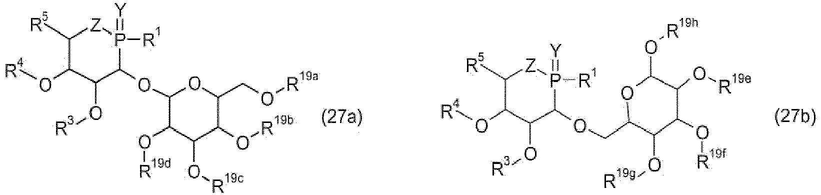

Une autre classe de composés (1) préférée correspond aux composés suivants de formule (24a), dans laquelle les différents substituants ont les significations indiquées précédemment. Parmi ces composés, ceux de formules (24b) et (24c) sont préférés :

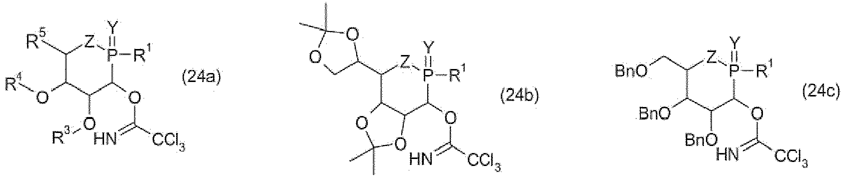

Les composés (1) dans lesquels R¹ est un atome d'hydrogène, notés composés (28), et les composés (30) dans lesquels R²¹ représente un groupe aryle sont également des composés préférés, les groupes aryle pouvant représenter des groupes aussi divers que les groupes phényle, 2,4-difluorophényle, 3,4-difluorophényle, 4-méthoxyphényle, 3,4-dinitrophényle, 4-nitrophényle :

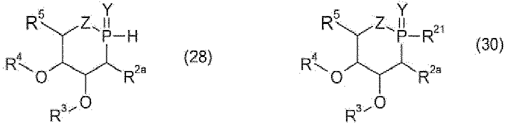

Bien que les phosphinosucres (1) selon l'invention puissent être obtenus sous une forme dans laquelle leurs groupes hydroxyle sont protégés, l'invention n'est nullement limitée à ce mode de réalisation et englobe également les phosphinosucres (1) obtenus sous une forme dans laquelle tous leurs groupes hydroxyle sont déprotégés, ou seulement certains d'entre eux. Les phosphinosucres polyhydroxylés préférés correspondant à cet autre mode de réalisation de l'invention, ainsi que d'autres phosphinosucres préférés, répondent aux formules ci-dessous, dans lesquelles R¹, R^{2a}, Y et Z ont la signification indiquée précédemment, R²² désigne un groupe CF₃ ou un groupe aryle, par exemple le groupe 4-tolyle (R^{2a} représentant de préférence un groupe OSO₂R²² tel que défini ci-dessus, un groupe N₃ ou un atome d'halogène, de préférence un atome de fluor), et R³⁰ et R³¹ désignent indépendamment l'un de l'autre un groupe hydrocarboné ou un atome d'hydrogène :

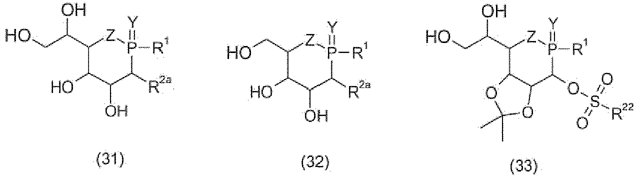

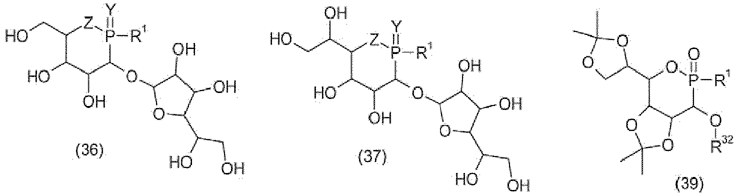

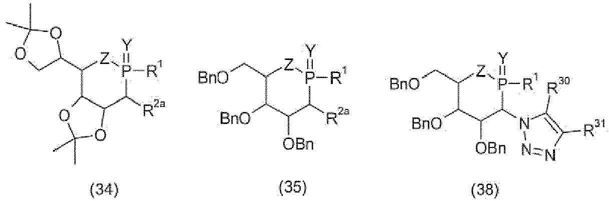

Dans la formule (39), R¹ a la signification indiquée précédemment et R³² est choisi parmi les mêmes substituants que ceux indiqués précédemment pour le groupe R¹⁹, R³² représente de préférence un groupe acyle, notamment le groupe benzoyle. Dans la formule (38), R³⁰ et R³¹ représentent de préférence indépendamment l'un de l'autre un groupe alkyle, aryle ou un atome d'hydrogène. On préfèrera les composés de formule (38) dans lesquels l'un des groupes R³⁰ et R³¹ est un atome d'hydrogène, et l'autre est un groupe hydrocarboné, mieux ceux dans lesquels R³⁰ = H et R³¹ = aryle, idéalement phényle. Les composés de formule (38), comportant un substituant 1H-[1,2,3]-triazolyle, peuvent notamment être obtenus par réaction des azotures correspondants avec un alcyne (par exemple par cycloaddition azoture-alcyne catalysée par le cuivre(I)). Le composé de formule (38) préféré a la structure suivante (38a) :

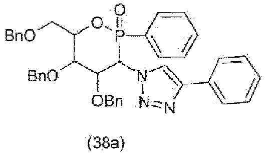

L'un des composés de formule (35) préférés a la structure suivante (35a) :

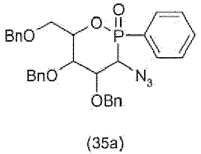

Les composés de formule (1) dans lesquels R^{2a} est un atome d'halogène (de préférence fluor, chlore ou brome) peuvent être obtenus à partir du triflate correspondant (R^{2a} = OSO₂CF₃, lui-même obtenu à partir du composé dans lequel R^{2a} = OH), par substitution nucléophile en présence d'un sel d'halogénure approprié. On peut notamment utiliser les sels d'ammonium n-Bu₄N⁺X (X = F, Cl, Br, I). Il est également possible de convertir directement les composés de formule (1) dans lesquels R^{2a} = OH en dérivés halogénés correspondants par réaction avec le réactif de Castro (Ph₃PX₂ avec X = Cl, Br, I) ou d'Appel (Ph₃P, CX₄ avec X = Cl, Br, I).

Les classes suivantes de composé de formule (1) sont préférées : ceux ayant un noyau aromatique sur l'atome de phosphore, et/ou les dérivés du mannose ayant un hétéroatome substitué en position C1 en α de l'atome de phosphore, et/ou ceux ayant une protection des groupes hydroxyle en position non anomérique.

Selon un mode de réalisation, les composés suivants sont exclus des produits et/ou méthodes de l'invention :

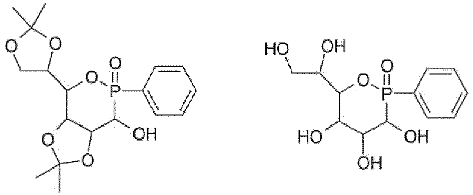

### Compositions comprenant un composé de formule (1) selon l'invention

Comme cela a déjà été mentionné précédemment dans la présente description, les composés de formule (1) possèdent, outre une activité anticancéreuse antiproliférative, une activité antimétastatique dans le sens où ils inhibent ou réduisent la migration de cellules cancéreuses, et constituent donc des principes actifs utilisables dans tout type de composition anticancéreuse.

L'invention est relative à un composé de formule (1) tel que défini dans la présente description, pour son utilisation en tant que médicament à usage humain ou vétérinaire, pour réduire ou prévenir l'apparition de métastases chez un patient atteint d'un cancer.

En particulier, les composés de formule (1) sont utiles en tant que principes actifs dans des compositions pharmaceutiques pour usage humain ou vétérinaire, destinées au traitement de cancers (métastatiques ou primaires), c'est-à-dire de cellules cancéreuses, ou à la prévention de l'apparition de cancers (par exemple les composés de formule (38)), notamment pour réduire ou prévenir l'apparition de métastases chez un patient atteint d'un cancer. Dans le cas où le patient est atteint d'un cancer métastatique, les composés de formule (1) visent notamment en particulier à réduire ou prévenir l'apparition de métastases supplémentaires.

Dans la présente description, un patient désigne aussi bien un animal, en particulier un mammifère non humain, qu'un homme. Par patient atteint d'un cancer, on entend aussi bien un patient atteint d'un cancer (primaire ou métastatique) déclaré que d'un cancer occulte, c'est-à-dire invisible, dont on a eu par exemple connaissance de l'existence par la découverte de métastases.

L'invention a encore pour objet une composition pharmaceutique pour un usage en médecine humaine ou vétérinaire comprenant au moins un composé de formule (1) tel que défini dans la présente description, de préférence en association avec un ou plusieurs excipients et/ou véhicules pharmaceutiquement acceptables, en particulier pour réduire ou prévenir l'apparition de métastases chez un patient atteint d'un cancer.

Dans la présente demande, les cellules cancéreuses désignent des cellules possédant des caractéristiques typiques de cellules provoquant le cancer, telles qu'une prolifération incontrôlée, immortalité, potentiel métastatique, croissance rapide et grande vitesse de prolifération, et certaines caractéristiques morphologiques spécifiques. Les cellules cancéreuses se présentent souvent sous la forme d'une tumeur, mais de telles cellules peuvent exister seules à l'intérieur du corps, ou peuvent être des cellules cancéreuses non tumorigènes, telles que des cellules leucémiques. Les cellules cancéreuses peuvent être associées à de nombreux types de cancers, comprenant sans limitation, la leucémie, un lymphome, un mélanome, un neuroblastome, le cancer du foie, des ovaires, du cerveau, du poumon, du colon, du sein, du pancréas, de la prostate, du testicule, de l'œsophage, de l'utérus, du col de l'utérus, du rein, de l'estomac, de la vessie, un cancer cérébrospinal, un cancer colorectal. Les compositions pharmaceutiques de l'invention peuvent être utilisées pour le traitement thérapeutique d'au moins l'un des cancers cités ci-dessus.

Lorsque les composés selon l'invention sont utilisés dans le cadre d'un traitement antimétastatique, le patient est atteint d'un cancer dit primaire. Ce cancer est un cancer capable de métastaser, qui peut être, sans limitation, un mélanome, un glioblastome multiforme, un cancer du poumon, notamment cancer du poumon non à petites cellules, un cancer du côlon ou colorectal, du sein, de la prostate, du testicule, du col de l'utérus, du rein, de préférence un glioblastome multiforme, un cancer du sein ou un cancer du poumon non à petites cellules. Les composés de l'invention sont particulièrement adaptés pour le traitement du risque métastatique chez un patient atteint d'un glioblastome multiforme. Il est maintenant reconnu que le glioblastome multiforme (GBM), couramment nommé glioblastome, peut être un cancer à potentiel métastatique donnant lieu à une pathologie généralisée (Schönsteiner, S. S. et al., Journal of Clinical Oncology 2011, 29, 23, 668-671). Les cellules cancéreuses issues de glioblastomes peuvent effectivement passer au travers de la barrière hématoencéphalique et établir des métastases extraneurales. Les sites reportés de métastases extraneurales sont les poumons, la plèvre, le foie, les nodules lymphatiques cervicaux, les os et la moelle osseuse.

Une composition pharmaceutique humaine ou vétérinaire selon l'invention peut aussi comprendre un ou plusieurs autres principes actifs différents d'un composé de formule (1), notamment pour augmenter son efficacité, y compris un ou plusieurs autres composés anticancéreux (antiprolifératifs et/ou antimétastatiques). Comme autres principes actifs susceptibles d'être inclus dans une composition pharmaceutique selon l'invention, on peut citer notamment des agents antihistaminiques, des agents anti-inflammatoires, des agents désinfectants ou encore des agents anesthésiques locaux.

Selon un mode de réalisation de l'invention, la composition selon l'invention contient uniquement comme principes actifs anticancéreux des composés de formule (1).

Selon un autre mode de réalisation de l'invention, la composition selon l'invention contient un agent anticancéreux additionnel, tel qu'un taxane (par exemple le paclitaxel ou le docétaxel), la vinblastine, la vinorelbine, la vincristine, la bléomycine, le témozolomide, le 5-fluorouracile et/ou un inhibiteur d'angiogenèse (par exemple le bevacizumab).

Alternativement ou additionnellement, la composition selon l'invention peut être utilisée en association avec un autre traitement thérapeutique, notamment en association avec un traitement anticancéreux additionnel administré séparément, tel qu'un traitement au moyen de l'un des anticancéreux additionnels cités ci-dessus, ciblant par exemple la prolifération des cellules cancéreuses et/ou l'angiogenèse.

Ainsi, l'invention concerne également un produit de combinaison pour une utilisation simultanée, séparée ou étalée dans le temps, pour le traitement d'un cancer, comprenant au moins un composé cytotoxique (destiné au traitement de la prolifération dudit cancer), et au moins un composé de formule (1) selon l'invention pour réduire ou prévenir l'apparition de métastases.

Une composition pharmaceutique comprenant un composé anticancéreux selon l'invention se présente indifféremment sous une forme solide (particules sèches) ou sous une forme liquide. Sous la forme liquide, on préférera une composition pharmaceutique sous la forme d'une suspension aqueuse ou d'une suspension non aqueuse, ou encore sous la forme d'une émulsion eau-dans-huile ou huile-dans-eau.

Parmi les compositions pharmaceutiques selon l'invention, on pourra citer plus particulièrement celles qui conviennent pour l'administration orale, topique, parentérale, nasale, intraveineuse, percutanée (transcutanée), sous-cutanée, rectale, perlinguale ou respiratoire et notamment les comprimés simples ou dragéifiés, les comprimés sublinguaux, les gélules, les tablettes, les suppositoires, les crèmes, les pommades, les gels dermiques, et les ampoules buvables ou injectables.

La posologie varie selon le sexe, l'âge et le poids du patient, selon la voie d'administration, et selon le type de cancer, l'état de progression du cancer, en particulier selon que des métastases ont été détectées ou non chez le patient. La posologie peut également varier selon le type du ou des traitement(s) anti-cancéreux associé(s).

De manière générale, on utilise un composé de formule (1) tel que défini dans la présente description dans des quantités allant de préférence de 0,001 mg/kg de poids corporel du patient ou de l'animal, à 1 g/kg de poids corporel du patient ou de l'animal par 24 heures, en une ou plusieurs prises. De préférence, ladite quantité est au moins égale à 0,01 mg/kg, mieux 0,05 mg/kg. De préférence, ladite quantité est au plus égale à 500 mg/kg, mieux 100 mg/kg.

Pour une administration par voie orale, une composition pharmaceutique selon l'invention peut de présenter sous la forme de comprimés, de gélules, de capsules, de dragées, de sirops, de suspensions, de solutions, de poudres, de granulés, d'émulsions, de suspensions de microsphères ou de nanosphères, de suspensions de vésicules lipidiques ou de vésicules à base de divers polymères.

Pour une administration orale, une composition pharmaceutique selon l'invention peut se présenter sous la forme de comprimés qui peuvent être fabriqués à partir de compositions solides comprenant au moins un composé de formule (1) en combinaison avec divers excipients tels que la cellulose microcristalline, le citrate de sodium, le carbonate de calcium, le phosphate dicalcique, ou la glycine. On peut utiliser divers agents désintégrants tels que l'amidon (de maïs, de pommes de terre, de tapioca, etc.), de l'acide alginique ou encore un silicate. On peut utiliser des agents liants tels que la polyvinylpyrrolidone, le saccharose, la gélatine, ou encore l'acacia. On peut utiliser des agents lubrifiants tels que le stéarate de magnésium, le lauryl sulfate de sodium, ou encore le talc. De telles compositions solides, sous forme d'une poudre, peuvent être utilisées pour la fabrication de capsules de gélatine. Pour des compositions solides, on peut aussi utiliser du lactose ou encore un polyéthylène glycol de haut poids moléculaire.

Pour fabriquer des compositions liquides pour administration orale, le composé de formule (1) peut être combiné avec divers agents édulcorants, agents d'arôme, agents colorants, éventuellement avec également des agents émulsifiants ou des agents de suspension, en combinaison avec des agents diluants tels que l'eau, l'éthanol, un propylène glycol, de la glycérine ou toute combinaison de ces agents excipients.

Pour une administration par voie parentérale, une composition pharmaceutique selon l'invention peut se présenter sous la forme de solutions ou de suspensions pour perfusion ou injection.

Pour une administration par voie parentérale, on peut utiliser en particulier des solutions huileuses ou aqueuses ou encore des suspensions, des émulsions, ou des implants y compris des suppositoires. Par exemple, un composé de formule (1) peut être dispersé dans un véhicule liquide tel qu'un liquide salin physiologique ou encore une solution saline contenant 5% en poids de dextrose, qui sont classiquement utilisées pour la préparation de formulations pharmaceutiques injectables.

Pour une administration par voie entérale, on peut utiliser des compositions à libération contrôlée, par exemple des compositions dans lesquelles le composé de formule (1) est protégé du milieu extérieur par une pluralité de couches de revêtement qui se dégradent de manière différente, par exemple au contact d'un milieu neutre ou basique (couches de revêtement gastro-résistantes) ou au contact d'un milieu aqueux (couches de revêtement comprenant des polymères solubles ou qui se dégradent dans l'eau).

La composition pharmaceutique de la présente invention peut être utilisée pour une administration parentérale, topique ou locale et de manière prophylactique et/ou thérapeutique. Ainsi le composé anticancéreux selon la présente invention est préparé sous une forme adaptée au type d'administration choisi, par exemple sous forme liquide ou sous forme lyophilisée.

Les compositions pharmaceutiques comprenant un composé anticancéreux selon l'invention peuvent contenir un excipient et/ou un véhicule pharmaceutiquement acceptable, liquide ou solide, par exemple aqueux. De nombreux excipients et/ou véhicules pharmaceutiquement acceptables peuvent être utilisés, par exemple des solvants ou diluants ; l'eau, le cas échéant en mélange avec du propylène glycol ou du polyéthylène glycol, l'eau tamponnée, une solution saline, une solution de glycine et ses dérivés, une solution non aqueuse comprenant notamment des solvants tels que l'éthanol, la N-méthylpyrrolidone, le diméthylacétamide (DMA), le diméthylsulfoxyde (DMSO) et/ou le diméthyl formamide (DMF), ainsi que des agents nécessaires pour reproduire les conditions physiologiques comme par exemple des agents tampons et ajusteurs de pH, des surfactants comme le Solutol® HS15, le Tween® 80, l'acétate de sodium, le lactate de sodium, le chlorure de sodium, le chlorure de potassium, le chlorure de calcium, ou un véhicule tel que le Cremophor EL®, cette liste n'étant pas limitative. De plus, la composition pharmaceutique peut être stérilisée par des techniques de stérilisation bien connues de l'homme du métier. On préfère utiliser au moins un solvant organique et au moins un surfactant pour solubiliser les composés de formule (1), de préférence un mélange d'éthanol, de diméthylacétamide et d'au moins un surfactant.

En tant que véhicules, adjuvants ou excipients inertes, non toxiques, pharmaceutiquement acceptables, on peut aussi citer à titre indicatif et non limitatif les agents solubilisants autres que les solvants, les conservateurs, les agents mouillants, les émulsifiants, les agents dispersants, les liants, les agents gonflants, les agents désintégrants, les agents d'encapsulation, les retardants, les lubrifiants, les absorbants, les agents de suspension, les colorants, les arômes, les stabilisants, les agents épaississants etc. De tels composés sont par exemple le carbonate de magnésium, le stéarate de magnésium, le talc, le lactose, la pectine, la dextrine, l'amidon, la gélatine, des matériaux cellulosiques, du beurre de cacao, etc.

De manière générale, une composition pharmaceutique selon l'invention comprend de 0,01% à 99% en poids, et avantageusement de 1% à 90% en poids, de composés de formule (1), par rapport au poids total de ladite composition. La dose de composés (1) administrée quotidiennement varie généralement de 0,5 à 50 mg/kg, de préférence de 1 à 20 mg/kg. De manière générale, une composition pharmaceutique selon l'invention comprend de 1% à 99,99% en poids, et avantageusement de 10% à 99% en poids, d'un excipient et/ou véhicule (ou diluant) ou d'une combinaison d'excipients et/ou véhicules pharmaceutiquement acceptables.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues.

On peut enrober les comprimés de saccharose ou d'autres matières premières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une composition pharmaceutique sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants ou des agents de mise en suspension, comme la polyvinylpyrrolidone, de même qu'avec des édulcorants ou des correcteurs de goût.

Le principe actif peut être formulé également sous forme de microcapsules, éventuellement avec un ou plusieurs supports ou additifs.

De manière générale, pour fabriquer une composition pharmaceutique conforme à l'invention, l'homme du métier peut avantageusement se référer à la dernière édition de la Pharmacopée Européenne, par exemple à la 5ème édition de la Pharmacopée Européenne publiée en janvier 2005, ou bien encore à la 6è édition de la Pharmacopée Européenne, disponible au public en juin 2007.

Des techniques de préparation de compositions pharmaceutiques selon l'invention peuvent être aisément retrouvées par l'homme du métier, par exemple dans l'ouvrage Remmingston's Pharmaceutical Sciences, Mid. Publishing Co, Easton, PA, USA.

Des véhicules et des excipients (ou adjuvants) physiologiquement acceptables sont aussi décrits dans l'ouvrage intitulé "Handbook of Pharmaceutical Excipients," Seconde édition, American Pharmaceutical Association, 1994.

Pour formuler une composition pharmaceutique selon l'invention, l'homme du métier pourra avantageusement se référer à la dernière édition de la Pharmacopée Européenne ou de la Pharmacopée des Etats-Unis d'Amérique (U.S. Pharmacopeia, notamment l'édition USP 30-NF 25).

Avantageusement, une composition pharmaceutique telle que définie ci-dessus est adaptée pour une administration orale, parentérale ou intraveineuse.

Lorsque la composition pharmaceutique selon l'invention comprend au moins un excipient pharmaceutiquement ou physiologiquement acceptable, il s'agit en particulier d'un excipient approprié pour une administration de la composition par voie orale ou d'un excipient approprié pour une administration de la composition par voie parentérale.

L'invention concerne aussi une méthode de traitement thérapeutique, pour prévenir ou traiter le développement d'un cancer chez un patient, et/ou pour réduire ou prévenir l'apparition de métastases chez un patient atteint d'un cancer, ladite méthode comprenant une étape au cours de laquelle on administre au patient une quantité thérapeutiquement efficace d'un composé de formule (1) tel que défini dans la présente description ou encore d'une composition pharmaceutique contenant ledit composé de formule (1).

Le traitement antimétastatique selon l'invention est généralement un traitement anticancéreux complémentaire ou adjuvant d'un traitement principal. Ce traitement adjuvant s'applique généralement dans un deuxième temps quand, à la suite du traitement initial, on recueille des informations incitant à un supplément thérapeutique. Toutefois dans certains cas, ce traitement adjuvant peut être appliqué en premier, lorsque les indications en faveur de ce traitement complémentaire sont connues d'emblée. Si les analyses effectuées accréditent la thèse d'une dissémination microscopique dans l'organisme, un traitement général par chimiothérapie au moyen des composés selon l'invention se justifie pour traiter cette généralisation avant que ne se manifestent des métastases.

L'invention peut être mise en œuvre en combinaison avec d'autres modalités de traitements, telles que l'hormonothérapie, la chirurgie, la cryothérapie, l'hyperthermie, la radiothérapie, une chimiothérapie additionnelle, etc. Avantageusement, le traitement selon l'invention peut être associé avec un traitement ciblant la prolifération des cellules cancéreuses et/ou l'angiogenèse.

L'invention est en outre illustrée, sans pour autant être limitée, par les exemples suivants.

### EXEMPLES

### a) Matériel

Sauf indication contraire, les spectres RMN ont été enregistrés sur un spectromètre BRUKER AVANCE 400 travaillant à 400,13 MHz (¹H) dans le CDCl₃. Les déplacements chimiques sont exprimés en ppm/TMS pour ¹H et ¹³C; les constantes de couplage ⁿJ sont exprimées en Hz. Quand les spectres sont du premier ordre, ou peuvent être considérés comme tels, les signaux sont désignés par les lettres s (singulet), d (doublet), t (triplet), q (quadruplet), m (multiplet) et par combinaisons de ces lettres. Un signal élargi sera désigné par une de ces lettres, suivie de la lettre l.

Les spectres de masse à haute résolution (MS HR) ont été enregistrés sur un JEOL JMS DX-300 en mode d'ionisation FAB positive avec comme matrice l'alcool *p*-nitrobenzylique (NBA).

Les composés sensibles à l'humidité ou à l'oxygène ont été manipulés sous azote en utilisant les techniques de Schlenk. Les solvants anhydres sont distillés sous azote avec l'agent desséchant approprié. La séparation des produits par chromatographie flash est réalisée sur colonne de gel de silice Merck 15-40 µm ou 30-75 µm.

Des souris nude femelles de 4-5 semaines ayant un poids moyen de 25 mg fournies par Centre d'Elevage R. Janvier ont été utilisées. Les souris ont été maintenues dans un environnement sans pathogène et nourriture et eau présents *ad libitum.* Les expériences ont commencé après une semaine d'acclimatation des souris à leur nouvel environnement.

Plusieurs lignées de cellules cancéreuses ont été utilisées pour les tests biologiques in vivo ou in vitro :
- SNB75 est une lignée de glioblastome humain dérivée d'une culture primaire, c'est-à-dire issue d'une tumeur cancéreuse maligne primitive. Elle fait partie du panel des 59 lignées cancéreuses référencées par le NCI. Son numéro de référence COSMIC publié par le Welcome trust Sanger Institute est 905982. Cette lignée a été fournie par par Mr Souhgheng Ning (M.D. Ph.D. du département de « Radiation Oncology », Stanford University Medical center). Elle a été cultivée dans du Ham's F-12 and DMEM (1/1) supplémenté avec 10% de sérum bovin foetal. Les expériences ont été réalisées avec des cellules en phase exponentielle de croissance.
- Gli4 (ou Gli4F11) et Gli7 sont des cultures de cellules souches cancéreuses issues de glioblastomes humains (cultures primaires). Ces lignées sont CD133⁺, CD15⁺ multipotentes et tumogéniques chez l'animal. Elles ont été établies par J. P. Hugnot (INM INSERM U-1051 Eq. 4), et décrites dans Guichet, P. O. et al, Glia 2013, 61(2), 225-239.
- C6 est une lignée de glioblastomes de rat. Elle est référencée à l'ATCC sous la référence CCL-107.
- MDAMB-435 est une lignée de mélanome issue d'une métastase mammaire. Elle fait partie du panel des 59 lignées cancéreuses référencées par le NCI. Elle est référencée à l'ATCC sous la référence HTB-129. Elle a été cultivée dans du Ham's F-12 and DMEM (1/1) supplémenté avec 10% de sérum bovin fœtal. Les expériences ont été réalisées avec des cellules en phase exponentielle de croissance.

### b) Préparation de composés de nature oxazaphosphinane

### b.1) Composé 3.48a (4,5-bis-benzyloxy-6-benzyloxymethyl-2-phényl-2-oxo-2λ⁵-[1,2] oxaphosphinan-3-ol)

Dans un tricol sous azote muni d'un réfrigérant, on introduit successivement 500 mg (0,9 mmol) du mélange de diastéréomères de formule **3.47** comportant une liaison P-H décrit dans la demande WO 2009/004096, 125 mg de palladium tétrakis(triphénylphosphine) (0,09 mmol, 0,1 éq), 5 mL de toluène anhydre, l'iodobenzène ou le bromobenzène (0,9 mmol, 1 éq) et 365 µL de triéthylamine (2,7 mmol, 3 éq). Le milieu réactionnel est porté à 70°C durant 4h sous agitation magnétique. Le milieu réactionnel est filtré sur célite, le filtrat ainsi obtenu est évaporé sous pression réduite. Le résidu huileux est alors purifié par chromatographie sur gel de silice (gradient Ch₂Cl₂/AcOEt 100/0 à 50/50), permettant de séparer deux diastéréomères **3.48a** et **3.48b** obtenus dans un ratio molaire 81/19. On obtient le diastéréoisomère **3.48a** sous la forme d'un solide incolore avec un rendement global de 60 % (C₃₂H₃₃O₆P, M = 544,59 g/mol). Une méthode plus efficace pour la préparation de ce composé a été décrite dans la publication J. Med. Chem. 2012, 55, 2196-2211. 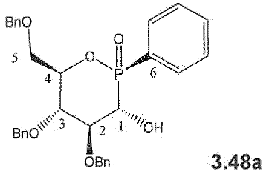 **¹H RMN (400,13 MHz, CDCl₃)** : δ 3,68 (dd, 1H, ³J_{H-H} = 1,9 Hz, ²J_{H-H} = 11,1 Hz, **⁵*CH*_{*2*a}**), 3,86-3,91 (m, 2H, ***⁵CH_{zb}*+ *⁴CH),*** 3,94 (dl, 1H, ²J_{P-H} = 9,6 Hz, ***¹CH***), 4,11 (m, 1H, ***³CH***), 4,44-4,63 (m, 4H, 2 ***CH₂OBn**),* 4,80-4,84 (m, 2H, ***CH₂OBn***), 4,89 (dd, 1H, ³J_{P-H} = 11,3 Hz, ³J_{H-H} = 4,7 Hz, ***²CH***), 7,12-7,74 (m, 20H, ***CH_{Ar}**).*
MS **HR⁺** (NBA) : Masse théorique 545,2093 ; Masse expérimentale 545,2104.

### b.2) Composé 4.2a (S_{P}RSRR)-3-benzoate-4-(2.2-diméthyl-[1.3]dioxolan-4-yl)-2.2-diméthyl-2-oxo-2-phényl-tétrahydro-2λ⁵-[1.2]oxaphosphinane)

Sous atmosphère d'azote, de l'anhydride benzoïque (132 mg, 0.58 mmol), de la pyridine (25 µl, 0.39 mmol) et de la DMAP (4.8 mg, 0.039 mmol) sont ajoutés à une solution du composé **3.3a** décrit dans la demande WO 2009/004096 (150 mg, 0.39 mmoles) dans du CH₂Cl₂ anhydre (3 mL). Le mélange réactionnel est agité à température ambiante durant une nuit. La solution organique est lavée à l'eau et par une solution aqueuse saturée de chlorure de sodium. Les phases organiques combinées sont séchées sur sulfate de sodium, filtrées et concentrées sous vide. Le résidu est dissous dans la quantité minimale de CH₂Cl₂ nécessaire et reprécipité dans l'hexane. On obtient le composé **4.2a** sous la forme d'un solide incolore avec un rendement global de 78 % (149 mg). 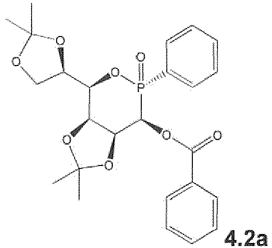 ³¹P NMR (161.97 MHz, DMSOd₆): δ = 25.02 ppm. ¹H RMN (400.13 MHz, DMSO d₆): δ = 1.30 (s, 3H), 1.33 (s, 3H), 1.38 (s, 3H), 1.56 (s, 3H), 3.86 (dd, 1H, *J* = 5. 8 Hz), 4.06 (dd, 1H, *J* = 7. 8 Hz), 4.31 (dd, 1H, J = 12. 6 Hz), 4.70 (d, 1H, *J* = 8 Hz), 4.86 (d, 1H, *J* = 6 Hz), 4.98 (ddd, 1H, *J* = 24, 8, 4 Hz), 6.00 (d, 1H, J = 4 Hz), 7.53-7.97 (m, 10H) ppm. ¹³C RMN (100.61 MHz, DMSOd₆): δ = 24.39, 25.16, 25.62, 26.28, 65.48, 67.16 (d, *J* = 105.3 Hz), 73.41 (d, *J =* 8.05 Hz), 73.46 (d, *J* = 6 Hz), 73.93 (d, *J* = 8.05 Hz), 75.72 (d, *J* = 8.05 Hz), 108.79, 109.92, 128.29, 128.68 (d, *J* = 13.2 Hz), 129.18 (d, *J* = 51.2 Hz), 130.78 (d, *J* = 10.2 Hz), 131.28 (d, *J* = 134.7 Hz), 132.73 (d, *J* = 2.4 Hz), 134.04, 164.78 ppm. HRMS (ESI) *m*/*z* calculée pour C₂₅H₃₀O₈P (M+H)⁺: 489.1678; trouvé : 489.1669.

### b.3) Synthèse du 5,6-Di-O-isopropylidène-N-benzyl-D-mannosylamine

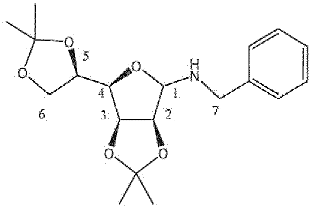

Dans un tricol, on introduit 10 g de D-mannose protégé ([2,3,5,6]-di-O-isopropylidène-D-Mannose, décrit dans la demande WO 2009/004096; 38 mmol), 100 mL d'éthanol, 10 g de sulfate de magnésium et 7,5 mL de benzylamine distillée (57 mmol , 1,5 éq). Le milieu est agité à reflux durant 48 heures. Le milieu est filtré sur célite, le filtrat obtenu est évaporé sous pression réduite. Le résidu huileux est directement purifié sur colonne de gel de silice avec un système dichlorométhane/acétate d'éthyle suivant un gradient de 100/0 à 50/50. Le produit est obtenu sous la forme d'une huile jaune. Rdt 58%.
**¹H RMN (400,13 MHz, CDCl₃)** : δ 1,19 (s, 3H, ***CH₃***), 1,24 (s, 3H, ***CH₃***), 1,30 (s, 3H, ***CH₃***), 1,38 (s, 3H, ***CH₃***), 3,30 (dd, 1H, ³J_{H-H} = 3,3 Hz, ³J_{H-H} = 7,6 Hz, ***⁴CH***), 3,67 (dd, 1H, ³J_{H-H} = 5,9 Hz, ³J_{H-H} = 7,2 Hz, ***⁵CH***), 3,81 (dd, 1H, ²J_{H-H} = 3,8 Hz, ³J_{H-H} = 2,0 Hz, ***²CH***), 3,97 (m, 2H, ***⁶CH₂***), 4,29 (td, 1H, ³J_{H-H} = 3,3 Hz, ***³CH***), 4,48 (ddd, 2H, ²J_{H-H} = 41,8 Hz, ³J_{H-H} = 6,3 Hz, ²J_{H-H} = 3,5 Hz, ***⁷CH₂***), 4,66 (dd, 1H, ³J_{H-H} = 5,1 Hz, ³J_{H-H} = 3,6 Hz, ***¹CH***), 4,81 (s, 1H, ***NH***)*,* 7,22-7,47 (m, 5H, ***CH_{Ar}***).

### Synthèse du (R_{P}SSRR)-4-(2,2-Diméthyl-[1,3]dioxolan-4-yl)-2,2-diméthyl-2-oxo-2-phényl-tetrahydro-6λ*5*-[1,3]dioxolo[4,5-d]oxaphosphinan-3-aminobenzyl.

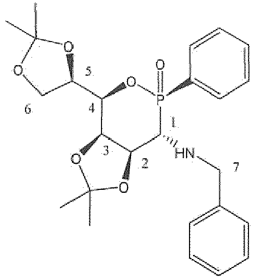

Dans un tube de Schlenk, désoxygéné au préalable et sous atmosphère d'azote, on place 1g du composé obtenu selon le paragraphe précédent (3 mmol), 10 mL de THF, 0,35 mL de phénylphosphinate de méthyle (3 mmol, 1 éq) et le tert-butylate de potassium (70 mg, 0,2 éq). Le milieu est ainsi agité à température ambiante durant 15 heures. 15 mL d'acide chlorhydrique 0,1N est alors additionné au milieu réactionnel, puis 15 mL d'une solution saturée en chlorure de sodium et 50 mL de chloroforme. La phase aqueuse est extraite par 2 fois 50 mL de chloroforme. Les phases organiques sont rassemblées, séchées sur sulfate de magnésium, et évaporées sous pression réduite. Le résidu huileux ainsi obtenu est alors purifié sur colonne de gel de silice en utilisant de l'éther diéthylique. Rendement 73%.
**³¹P RMN (161,97 MHz, CDCl₃**) : δ 38,31. **¹H RMN (400,13 MHz, CDCl₃)** : δ 1,35 (s, 3H, ***CH₃***), 1,37 (s, 3H, ***CH₃***), 1,40 (s, 3H, ***CH₃***), 1,45 (s, 3H, ***CH₃***), 3,35 (dd, 1H, ³J_{H-H} = 4,3 Hz, ²J_{P-H} = 7,6 Hz, ***¹CH***), 3,94 (sl, 2H, ***⁷CH₂***)*,* 4,14 (m, 2H, ***⁶CH₂***), 4,43 (m, 1H, ***³CH***), 4,49 (qd, 1H, ³J_{H-H} = 1,8 Hz, ³J_{H-H} = 7,6 Hz, ***⁴CH**),* 4,58 (m, 1H, ***⁵CH***), 4,69 (ddd, 1H, ³Jp-_{H} = 21,2 Hz, ³J_{H-H} = 4,3 Hz, ³J_{H-H} = 6,6 Hz, ***²CH***), 7,23-7,97 (m, 5H, ***CH_{Ar}**).* **¹³C RMN (100,61 MHz, CDCl₃)** : δ 24,66 (1s, 1C, ***CH₃***), 25,26 (1s, 1C, ***CH₃***), 26,31 (1s, 1C, ***CH₃***), 27,01 (1s, 1C, ***CH₃***), 52,97 (d, 1C, ¹J_{P-C} = 87,3 Hz, ***¹CH***), 53,05 (s, 1C, ***⁷CH2***), 66,81 (s, 1C, ***⁶CH₂***), 72,56 (d, 1C, ²J_{P-C} = 3,6 Hz, ***²CH***), 73,70 (d, 1C, ³J_{P-C} = 5,1 Hz, ***⁵CH***), 73,81 (d, 1C, ³J_{P-C} = 8,7 Hz, ***³CH***), 75,47 (d, 1C, ²J_{P-C} = 1,5 Hz, ***⁴CH***), 109,10 (s, 1C, ***Cq***), 110,01 (s, 1C, ***Cq***), 127,27 (s, 2C, ***CH_{ANI} m***), 128,14 (s, 2C, ***CH_{ANI}*** o), 128,79 (s, 1C, ***CH_{Ar} m***)*,* 128,30 (s, 1C, ***CH_{Ar} m***), 129,57 (d, 1C, J_{P-C} = 138,9 Hz, ***C_{q Ar}***), 129,60 (s, 1C, ***CH _{ANI} p***), 131,82 (s, 1C, ***CH_{Ar}*** o), 131,97 (s, 1C, ***CH_{Ar}* o**), 132,87 (s, 1C, ***CH_{Ar} p***), 138,94 (d, 1C, ³J_{P-C} = 17,4 Hz, ***Cq Aᵣ***). IR **(Pastille KBr)** : 3351 ff (v NH), 2980 ff, 2894 ff (v CH_{Alk}), 1491 ff, 1439 ff, 1370 f, 1265 f, 1205 FF, 1167 f, 1128 m (v C-O-C), 1059 f (v P = O), 962 m (v P-OC), 925 m (δ CH_{Ar}), 891 m, 817 f, 733 F, 713 f, 693 F. **MS FAB+ (NBA)** : m/z (%) 475 [MH+H ⁺] (90%) ; 474 [M+H ⁺] (50%). **MS HR⁺ (NBA)** : Masse théorique 474,2046 ; Masse expérimentale 474,2058.

### Synthèse du (R_{P}SSRR)-(2,2-Diméthyl-[1,3]dioxolan-4-yl)-2,2-diméthyl-2-oxo-2-phényl-tetrahydro-6λ*5*-[1,3]dioxo[4,5-d][1,2]oxaphosphinin-3-amino 5.5d

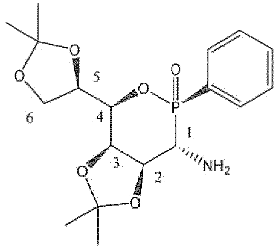

Dans un tube de Schlenk, désoxygéné au préalable, sous atmosphère d'azote, on place 1 g de phosphinosucre (0,002 mol), 10 mL d'éthanol, et 1 g de palladium sur charbon. Le milieu est ainsi agité à température ambiante durant 24 h sous une atmosphère d'hydrogène. Le milieu réactionnel est alors filtré sur célite afin d'éliminer le palladium. Le filtrat obtenu est évaporé sous pression réduite pour conduire à une huile légèrement colorée. Cette huile est purifiée sur colonne de gel de silice, le produit attendu ne sortant qu'en éluant à l'éthanol. Rendement 87%. **³¹P RMN** (161,97 MHz, CDCl₃) : δ 37,46. **¹H RMN** (400,13 MHz, CDCl₃) : δ 1,38 (s, 3H, ***CH₃***), 1,41 (s, 3H, ***CH₃***), 1,44 (s, 3H, ***CH₃***), 1,50 (s, 3H, ***CH₃***), 1,86 (sl, 2H, ***NH₂***), 3,49 (dd, 1H, ²J_{P-H} = 5,2 Hz, ³J_{H-H} = 6,4 Hz, ***¹CH***), 4,11 (m, 2H, ***⁶CH₂***), 4,39-4,58 (m, 4H, ***³CH*** + ***⁵CH***+ ***²CH*+ *⁴CH***), 7,39-7,87 (m, 5H, ***CH_{Ar}***). **¹³C** RMN (100,61 MHz, CDCl₃) : δ 25,04 (s, 1C, ***CH₃***), 25,27 (s, 1C, ***CH₃***), 26,93 (s, 1C, ***CH₃***), 26,95 (s, 1C, ***CH₃***), 48,78 (d, 1C, ¹J_{P-C} = 91,6 Hz, ***¹CH***), 66,78 (s, 1C, ***⁶CH₂***), 72,85 (d, 1C, ²J_{P-C} = 3 Hz, ***²CH***), 73,27 (d, 1C, ²J_{P-C} = 5,1 Hz, ***⁴CH***), 73,97 (d, 1C, ³J_{P-C} = 9,5 Hz, ***³CH***), 78,33 (s, 1C, ***⁵CH***), 109,23 (d, 1C, ***Cq***), 109,96 (d, 1C, ***Cq***), 128,71 (d, 1C, ²J_{P-C} = 138 Hz, ***Cq Ar***), 128,41 (s, 1C, ***CH_{Ar}***), 128,52 (s, 1C, ***CH_{Ar}***), 131,92 (s, 1C, *CHAr),* 132,02 (s, 1C, ***CH_{Ar}***), 133,02 (s, 1C, ***CH_{Ar} p***). **IR (Pastille KBr)** : 3287 f (v OH), 2987 ff, 2937 ff (v CH_{Alk}), 1593 ff (v NH), 1439 ff, 1372 f, 1207 FF (v P = O), 1159 m (v C-O-C), 1124 F (v C-O), 1060 FF, 958 F (v P-O-C), 920 f, 890 m, 836 m, 798 f, 727 F, 694 F. **MS FAB+ (NBA)** : m/z (%) 767 [2M + H⁺] (10%), 384 [M + H^{*}] (90%). **MS HR⁺ (NBA) :** Masse théorique 384,1576 ; Masse expérimentale 384,1567.

### Synthèse du composé 5.6d (R_{P}SSRR)-3-Acétamide-4-(2.2-diméthyl-[1.31dioxolan-4-yl)-2.2-diméthyl-2-oxo-2-phényl-tétrahydro-2λ-[1.2]oxaphosphinane

Sous atmosphère d'azote, de l'anhydride acétique (72 µL, 0.76 mmol) et de la triéthylamine (60 µL, 0.42 mmol) sont ajoutés à une solution du composé **5.5d** obtenu précedement (145 mg, 0.38 mmol) dans du CH₂Cl₂ anhydre (5 mL). Le mélange réactionnel est agité à température ambiante pendant 2h. La solution organique est lavée par une solution aqueuse saturée d'hydrogénocarbonate de sodium. Les phases organiques combinées sont séchées sur sulfate de sodium, filtrées et concentrées sous vide. Le résidu est alors purifié par chromatographie sur gel de silice (gradient heptane/AcOEt/EtOH 50/50/0 à 0/95/5). Le solide incolore obtenu est dissous dans la quantité minimale d'éther diéthylique nécessaire et reprécipité dans l'hexane. On obtient le composé **5.6d** avec un rendement global de 12 % (20 mg). 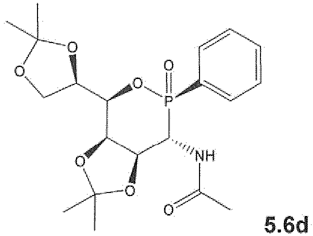 **³¹P RMN** (161,97 MHz, CDCl₃): δ = 37.02 ppm. **¹H RMN** (400,13 MHz, CDCl₃): δ = 1.31 (s, 3H), 1.34 (s, 3H), 1.37 (s, 3H), 1.53 (s, 3H), 1.91 (s, 3H), 4.03 (ddd, 2H, *J* = 4.5, 5.5, 9 Hz), 4.36-4.48 (m, 3H), 4.60-4.71 (m, 2H,), 6.74 (sl, 1H), 7.42-7.88 (m, 5H) ppm. **¹³C RMN** (100.61 MHz, CDCl₃): δ = 25.59, 26.41, 26.85, 27.51, 66.61, 68.01, 68.97, 74.72, 75.09, 75.17, 76.86, 76.93, 109.88, 111.04, 129.70, 130.03, 130.54, 131.92, 133.87, 135.50, 165.98. **HRMS (ESI)** *m*/*z* théorique pour C₂₀H₃₀NO₇P (M+H)^{*} : 426.1682; Masse expérimentale 426.1686.

### c) Formulation des composés de formule (1) selon l'invention

Les composés de formule **3.48a** et **4.2a** sont insolubles dans l'eau, dans de nombreux solvants hydrosolubles, et non complexables dans des cyclodextrines. Les deux formulations suivantes sont particulièrement adaptées aux composés **3.48a** et **4.2a** et permettent leur administration *in vivo,* à des concentrations de l'ordre de 20-100 mg/kg, par voie intraveineuse :
Formulation 1 : Solubilisation des composés **3.48a** et **4.2a** dans un mélange EtOH/DMA puis ajout extemporané de tensioactif Solutol® HS15 (Polyéthylène glycol-660-hydroxystéarate commercialisé par la société BASF) chauffé à 37°C et filtré (0,22 µm) pour obtenir un mélange EtOH/DMA/Solutol® HS15 (1/3/6 en volume), dilué en rajoutant 10% en volume d'une solution de NaCl 0,9%). La solution de NaCl 0,9% employée est une solution injectable (stérile et apyrogène), en flacons de verre stériles et à fermeture hermétique.
Formulation 2 : Préparée de la même façon que la formulation 1, pour aboutir à un mélange EtOH/DMA/Solutol® HS15 (1/3/6 en volume), dilué quatre fois avec du NaCl 0,9% (sauf lors de la préparation d'une formulation de composé **3.48a** ou **4.2a** à 5 mg/kg).

### d) Toxicité aiguë et chronique in vivo

Des études de toxicité aigüe des composés **3.48a** et **4.2a** ont été réalisées sur des souris saines pour une gamme de concentration de 50, 25, 10, 5 et 1 mg/kg. Aucune mortalité n'a été observée même pour la dose maximale de 50 mg/kg.

Une étude de toxicité chronique a été effectuée pour des injections de 20, 5 et 1 mg/kg à raison de 3 injections/semaine pendant 4 semaines. Ni mortalité, ni perte de poids n'ont été observées, même pour la dose maximale.

### e) Activité antimétastatique in vivo

### Modèle sous-cutané SNB75 (figure 1)

Une suspension de 5.10⁶ cellules de SNB75 dans 100 µL de PBS (phosphate Buffer saline, fourni par Fisher) stérile a été injectée à chaque souris (administration sous-cutanée sans anesthésie). Une fois que les souris ont présenté des tumeurs ayant atteint des volumes compris entre 0,13 et 0,033 mm³, des groupes ont été constitués de façon à ce que chacun d'entre eux contienne un nombre équivalent de grosses, moyennes et petites tumeurs. Le protocole a été mené avec 5 groupes de 7 souris. Les cinq groupes de souris ont été traitées au moyen de l'une des cinq formulations décrites ci-dessous :
Groupe A : Traitement par injection de 100 µL de NaCl (contrôle N° 1).
Groupe B : Traitement par le composé **3.48a** à raison de 10 mg/kg avec des volumes de 100 à 150 µL de formulation 2 selon le poids des souris.
Groupe C : Traitement par le composé **3.48a** à raison de 5 mg/kg avec des volumes de 100 à 150 µL de formulation 2 selon le poids des souris.
Groupe D : Traitement par le temozolomide à raison de 20 mg/kg avec des volumes de 100 à 150 µL selon le poids des souris. Le temozolomide est un principe actif anticancéreux comparatif commercialisé sous le nom de TEMODAL par le laboratoire Schering Plough. Il est indiqué pour traiter le glioblastome multiforme. La dose a été limitée à 20 mg/kg car une dose de 40 mg/kg entraîne le décès des souris avant la fin de la période d'expérimentation.
Groupe E : Traitement par injection de 100 à 150 µL selon le poids des souris de formulation 2 ne contenant pas de composé **3.48a** (contrôle N° 2).

Chaque lot a été traité par injection intraveineuse dans la veine caudale de 100 à 150 µL de solution (seringue de précision et aiguille G25X1 paroi ultrafine, VWR). 3 injections par semaine pendant 4 semaines ont été réalisées. Les souris ont été pesées à chaque injection. Les tailles des tumeurs ont été mesurées deux fois par semaine pendant une période de 4 semaines. Les souris ont été sacrifiées au bout de quatre semaine, les tumeurs et les organes (foie, rein, rate, cerveau, intestin, cœur, muscles) prélevés. Une analyse histologique a été réalisée sur ces organes.

Comme le montre la figure 1, une très forte réduction de la croissance de la tumeur primaire a été observée chez les souris traitées par le composé 3.48a (groupes B et C) au bout de 4 semaines, par rapport aux souris des groupes de contrôle (groupes A et E). Pour les groupes B et C, aucune des souris n'est décédée et aucune perte de poids n'a été observée. En ce qui concerne le lot D, 3 des 7 souris étaient décédées après la 3^{ème} injection, et des pertes de poids importantes ont été observées pour les 4 souris restantes, malgré une régression très importante de la tumeur.

La lignée cellulaire SNB75 provenant d'un glioblastome humain est capable de métastaser chez la souris nude suite à une implantation sous-cutanée. En effet, toutes les souris des groupes de contrôle (groupes A et E) ont développé des métastases au foie et à l'intestin. Aucune des souris traitées par le composé **3.48a** (groupes B et C) n'a développé de métastases. La figure 1A montre la présence de métastases au niveau du foie d'une souris du groupe A et l'absence de métastases au niveau du foie d'une souris du groupe B, 8 semaines après l'implantation sous cutanée des cellules SNB75.

### Modèle intracrânien Gli4

2.10⁵ cellules de Gli4 ont été injectées à un groupe de souris (injection striatale droite, coordonnées d'injection : bregma 0 L-L: L: -1,5mm P: -3,5-2,5). Les souris ont été immédiatement traitées pendant 4 semaines à raison de trois injections à 10 mg/kg de composé **3.48a** par semaine (formulation 1, groupe B) ou bien au moyen de 100 µL de NaCl selon le même protocole de traitement (groupe A) et sacrifiées au bout de 10 semaines.

Chez le groupe de souris témoin (groupe A), on observe des cellules dispersées de Gli4 qui ont migré le long du corps calleux et sont passées dans l'autre hémisphère. Les cellules Gli4 ont été identifiées avec un anticorps anti-noyau humain (anti-HuNu) révélé avec un anticorps secondaire anti-souris (Alexa 594). Cette migration reflète une migration le long de la substance blanche, comme observée dans les glioblastomes multiformes humains (dispersion selon une croissance intra fasciculaire le long de la substance blanche).

Chez le groupe de souris traité par le composé **3.48a** à raison de 10 mg/kg (groupe B), le traitement de quatre semaines a permis d'inhiber la migration cellulaire. Après l'arrêt du traitement, une reprise de la croissance et de la migration des cellules a été observée durant les six dernières semaines.

### Modèle sous-cutané MDAMB-435 (figures 4-6)

Une suspension de 1.10⁶ cellules de MDAMB-435 dans 100 µL de PBS (tampon phosphate salin, fourni par Fisher) stérile a été injectée à chaque souris (administration sous-cutanée, avec anesthésie, au niveau de la glande mammaire de la patte arrière gauche). Une fois qu'un pré groupe contrôle de 3 souris ont présenté les premières métastases (foie, intestin, ovaire) le protocole a été mené avec 4 groupes de 8 souris. Les cinq groupes de souris ont été traitées au moyen de l'une des cinq formulations décrites ci-dessous :
Groupe A : Traitement par injection de 100 µL de NaCl (contrôle N° 1).
Groupe B: Traitement par injection de 100 à 150 µL selon le poids des souris de formulation 2 ne contenant pas de composé **4.2a** (contrôle N° 2).
Groupe C : Traitement par le composé **4.2a** à raison de 10 mg/kg avec des volumes de 100 à 150 µL de formulation 2 selon le poids des souris.
Groupe D : Traitement par le composé **4.2a** à raison de 5 mg/kg avec des volumes de 100 à 150 µL de formulation 2 selon le poids des souris.

Chaque lot a été traité par injection intraveineuse dans la veine caudale de 100 à 150 µL de solution (seringue de précision et aiguille G25X1 paroi ultrafine, VWR). 3 injections par semaine pendant 4 semaines ont été réalisées. Les souris ont été pesées à chaque injection. Les souris ont été sacrifiées au bout de quatre semaine, les tumeurs et les organes (foie, rein, rate, cerveau, intestin, cœur, muscles) prélevés. Une analyse histologique a été réalisée sur ces organes.

La lignée cellulaire MDAMB-435 provenant d'un mélanome humain est capable de métastaser chez la souris nude suite à une implantation sous-cutanée au niveau des glandes mammaires. En effet, toutes les souris des groupes de contrôle (groupes A et B) ont développé des métastases au foie et à l'intestin. Les souris traitées par le composé **4.2a** (groupes C et D) ont vu une réduction du développement métastatique (métastases de petites tailles) et une forte réduction du nombre de métastase.

La figure 4 montre la présence de métastases aux ovaires chez les souris témoins (groupes A et B), et l'absence de métastase sur les souris traitées par le composé **4.2a** (groupes C et D).

La figure 5 présente le nombre moyen de métastases par souris en fonction des conditions de traitement. Elle montre qu'aucune des souris traitées par le composé **4.2a** (groupes C et D) n'a développé plus de deux métastases. En revanche, les souris témoins (groupes A et B) ont majoritairement développé plus de deux métastases.

La figure 6 présente la répartition de la masse de ces tumeurs secondaires en fonction des conditions de traitement. Elle montre que le traitement par le composé **4.2a** a réduit le développement des métastases.

### f) Activité antiprotiférative in vitro

Les lignées MDAMB-435 et A431 sont ensemencées à une densité de 3000 cellules par puits dans des plaques à 96 puits. Après 24h les composés **4.2a** et **5.6d** resuspendus en DMSO 100% sont ajoutés à des concentrations de 0 - 0,001 - 0,010 - 0,1- 1- 10- 50 µM pour une quantité finale constante en DMSO de 0,3 %. Les cultures sont maintenues 72 h supplémentaire puis un test colorimétrique MTT est réalisé. 100 µL d'une solution de MTT à 5mg/mL en PBS sont ajoutés au milieu de culture et laissés incuber pour 3 heures. Le milieu de culture est prélevé puis les cellules sont lysées par ajout de 100 µL d'un solvant 4 mM HCl, 0.1% Nondet P-40 (NP40) en isopropanol. Le sel jaune de tétrazolium MTT (bromure de 3-(4,5-dimethylthiazol-2-yl)-2,5-diphenyl tétrazolium), lorsqu'il est réduit par la succinate déshydrogénase mitochondriale des cellules vivantes actives, forme un produit formazan bleu-violet. Le milieu passe alors du jaune au bleu-violacé. L'intensité de cette coloration à 590 nm est proportionnelle au nombre de cellules vivantes présentes lors du test et donne une indication sur l'activité métabolique de ces cellules.

Le tableau 1 donne les valeurs de concentrations pour 50% d'inhibition de prolifération (IC₅₀) pour les composés **4.2a** et **5.6d** sur des lignées issues de cancer du côlon (CaCo2) de la peau (A431, MDAMB-435, B16F10) du sein (MDAMB-431) de l'intestin (HuH7), de la prostate (DU145) et du cerveau (SNB75, Gli4, Gli7).

**Tableau 1 : Effet antiprolifératif des composés 4.2a et 5.6d sur diverses lignées de cellules cancéreuses**

| | | Lignées cellulaires | | | | | | | | | | |
|---|---|---|---|---|---|---|---|---|---|---|---|---|
| | Composés | A431 | CaCo2 | MDAMB-231 | MDAMB-435 | HuH7 | B16F10 | DU145 | C6 | SNB75 | GLi4 | Gli7 |
| IC₅₀ µM | 4.2a | 4.35 ±0.40 | > 100 | 1.16 ± 0.00 | 0.66 ± 0.00 | > 100 | 13.48 ± 0.03 | > 100 | 8.51 ± 0.00 | 9.54 + 0.00 | 6.81 + 0.00 | 2.71 ± 0.00 |
| | 5.6d | 0.13 ± 0.00 | 35.96 ± 0.02 | 23.17 ± 0.01 | 9.61 ± 0.03 | 21.95 ± 0.00 | 14.87 ± 0.01 | 31.16 ± 0.01 | 0.65 ± 0.00 | 0.65 ± 0.00 | 0.54 ± 0.00 | 0.50 ± 0.00 |

Le tableau 1 montre que les composés **4.2a** et **5.6d** ont une activité antiproliférative sur différentes lignées cancéreuses de différents tissus humains et de rat. En particulier, le composé **4.2a** inhibe la prolifération cellulaire de la lignée de mélanome humain MDAMB-435 avec une IC₅₀ de 0,66 µM et le composé **5.6d** inhibe la prolifération de la lignée A431 (cancer épidermoïde humain) avec une IC₅₀ de 100 nM.

### g) Activité anti-migratoire in vitro

Les effets des composés **3.48a, 4.2a** et **5.6d** sur la migration de cellules cancéreuses (lignée humaine de glioblastome SNB75 ou culture primaire de glioblastome humain Gli4F11 d'une part, MDAMB 435 et A431 d'autre part), ont été déterminés en réalisant un test classique de migration en chambre de Boyden (traitement des cellules pendant 24 heures).

Pour ce test, les composés ont été solubilisés in vitro et utilisés en culture dans 0,3 % de DMSO. L'intérieur des inserts a été recouvert par la protéine de la matrice extracellulaire étudiée (laminine-1, vitronectine ou fibronectine. Les cellules prétraitées à différentes concentrations et non traitées (témoins) ont été déposées dans les inserts recouverts de ladite protéine à raison de 50000 cellules/500µL/insert dans un milieu contenant 0,1% de BSA (albumine sérique bovine).

500 µL de milieu contenant 10% de sérum de veau fœtal ont alors été placés dans chaque puits d'une plaque Companion afin de permettre la formation d'un gradient chimiotactique de part et d'autre de la membrane poreuse. Après 24h de migration dans l'incubateur à 37°C, le surnageant a été enlevé et les inserts rincés au PBS sur chaque face. Les cellules ont été comptées au microscope (Zeiss Axiophot, grossissement X200). C'est un pourcentage de migration par rapport aux cellules non traitées qui est donné.

Le tableau 2 ci-dessous donne pour le composé **3.48a** les valeurs des constantes d'inhibition (Ki) de la migration d'une lignée de GBM humain (SNB75), de lignées de cellules souches de GBM (Gli4, Gli7) et d'une lignée de cancer du sein MDAMB231, en chambre de Boyden, sur différents supports de protéines de la matrice extracellulaire. Les tableaux 3 et 4 ci-dessous donnent pour les composés **4.2a** et **5.6d** les valeurs des constantes d'inhibition (Ki) de la migration d'une lignée de mélanome (MDAMB-435) ou d'une lignée de cancer épidermoïde (A431), en chambre de Boyden, sur différents supports de protéines de la matrice extracellulaire. L'effet inhibiteur dépend de la lignée et des cellules souches utilisées mais aussi du support protéique de la matrice extracellulaire utilisée.

**Tableau 2 : Effet anti-migratoire du composé 3.48a sur différentes lignées et cellules souches de glioblastome multiforme**

| Support | | Fibronectine | Laminine | Vitronectine |
|---|---|---|---|---|
| | | Constante d'inhibition Ki (nmol) | | |
| Lignées | SNB75 | **27** | **23 000** | **87** |
| | Gli4 | **1** | **11** | **5** |
| | Gil7 | **37** | 310 | 371 |

**Tableau 3 : Effet anti-migratoire du composé 4.2a sur la lignée de mélanome MDAMB-435**

| Support | Fibronectine | Laminine | Vitronectine |
|---|---|---|---|
| | Constante d'inhibition Ki (nmol) | | |
| Lignée MDAMB-435 | **33** | **34** | **61** |

**Tableau 4 : Effet anti-migratoire du composé 5.6d sur la lignée de cancer épidermoïde A431**

| Support | Fibronectine | Laminine | Vitronectine |
|---|---|---|---|
| | Constante d'inhibition Ki (nmol) | | |
| Lignée A431 | **8** | **13** | **20** |

Les figures 2 et 3 et le Tableau 2 montrent que le composé **3.48a** inhibe la migration cellulaire des lignées de GBM SNB75 et C6 et cellules souche de GBM (Gli4 et Gli7) sur différents substrats de matrice extracellulaire avec un effet dose dépendant du substrat utilisé (vitronectine, fibronectine ou laminine-1). On obtient notamment une diminution de 50 % de la migration des cellules cancéreuses sur fibronectine ou vitronectine pour une concentration en composé **3.48a** aussi faible que 1 à 100 nM (lignée SNB75).

Les tableaux 3 et 4 montrent que les composés **4.2a et 5.6d** inhibent respectivement la migration cellulaire des lignées MDAMB-435 et A431 avec un effet dose dépendant du substrat utilisé (vitronectine, fibronectine ou laminine-1). On obtient notamment une diminution de 50 % de la migration des cellules cancéreuses sur fibronectine ou vitronectine pour une concentration en composé **4.2a et 5.6d** aussi faible que 10 à 100 nM.

Les composés (1) selon l'invention ont un mode d'action différent du mode d'action des composés supposés antimétastatiques sur le marché. Sans vouloir être liés par une théorie, les inventeurs pensent que les composés (1) ciblent la glycosylation cellulaire en inhibant les glycosyltransférases catalysant l'ajout de N-acétylglucosamines en position béta 1,6 sur les groupes mannoses liés en alpha 1,6. Les conséquences de ces modifications sont une inhibition du potentiel migratoire de ces cellules sur différents supports de la matrice extracellulaire comme la fibronectine ou encore la vitronectine.

## Revendications

1. Composé pour une utilisation en vue de réduire ou prévenir l'apparition de métastases chez un patient atteint d'un cancer, répondant à la formule générale ci-après : 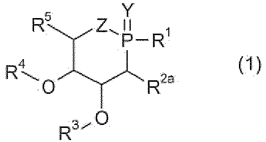 dans laquelle :
Y représente un atome d'oxygène, de soufre ou de sélénium, de préférence un atome d'oxygène,
Z représente O, S, Se, NH ou un groupe NR⁶, dans lequel R⁶ est un groupe aryle, hétéroaryle, alkyle ou cycloalkyle éventuellement substitué, de préférence un atome d'oxygène,
R¹ représente un atome d'hydrogène, un groupe alkyle ou cycloalkyle éventuellement substitué, ou un groupe aryle ou hétéroaryle,
R^{2a} représente un atome d'hydrogène, d'halogène, un groupe azoture (N₃), un groupe carbonate ou dithiocarbonate, un groupe 1H-[1,2,3]-triazolyle ou un groupe -X-R², X représentant un atome d'oxygène, de soufre, de sélénium, un groupe NH ou NR⁷, dans lequel R⁷ est un groupe aryle, hétéroaryle, alkyle ou cycloalkyle éventuellement substitué, X représentant de préférence O ou NH, et R² représentant un groupe aryle, hétéroaryle, alkyle ou cycloalkyle éventuellement substitué, un atome d'hydrogène, un groupe trichloroacétimidate (-C(=NH)CCl₃), acyle, formyle, sulfonyle, sulfinyle, *tert*-butyldiphénylsilyle, allyle, un groupe saccharyle, ester, amide, thioamide, sulfonamide, ou bien X-R² représente un groupe P(O)R^{2'}R^{6'} dans lequel R^{2'} et R^{6'} désignent, indépendamment l'un de l'autre, un groupe aryle, hétéroaryle, alkyle ou cycloalkyle éventuellement substitué, OH, alcoxy ou aryloxy,
R³ et R⁴ représentent, indépendamment l'un de l'autre, un groupe aryle, hétéroaryle, alkyle ou cycloalkyle éventuellement substitué, un atome d'hydrogène, un groupe trichloroacétimidate, acyle, formyle, sulfonyle, sulfinyle, *tert*-butyldiphénylsilyle, allyle, ester, amide, thioamide, sulfonamide, saccharyle, le groupe saccharyle dérivant de sucres ou de leurs analogues substitués ou déoxy, ou bien R³ et R⁴, pris conjointement, forment un radical bivalent de formule -R³-R⁴-, dans laquelle -R³-R⁴- représente de préférence un groupe isopropylydène, benzylidène, diphényl méthylidène, cyclohexyl méthylidène, éventuellement substitués, de préférence un groupe 4-méthoxybenzylidène, ou un groupe alkylène linéaire, de préférence le groupe éthylène,
R⁵ représente un atome d'hydrogène ou un groupe hydrocarboné comprenant un ou plusieurs hétéroatomes choisis de préférence parmi l'oxygène, le soufre ou l'azote, mieux l'oxygène.

2. Composé pour son utilisation selon la revendication 1, **caractérisé en ce que** Y = Z = O.

3. Composé pour son utilisation selon l'une quelconque des revendications 1 ou 2, **caractérisé en ce que** le groupe R⁵ est choisi parmi les groupes : 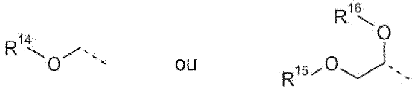 où R¹⁴, R¹⁵ et R¹⁶ représentent, indépendamment les uns des autres, un atome d'hydrogène, un groupe aryle, hétéroaryle, alkyle ou cycloalkyle éventuellement substitué, un groupe trichloroacétimidate, acyle, formyle, sulfonyle, sulfinyle, *tert*-butyldiphénylsilyle, allyle, ester, amide, sulfonamide, saccharyle ou bien R¹⁵ et R¹⁶, pris conjointement, forment un radical bivalent de formule -R¹⁵-R¹⁶-, dans laquelle -R¹⁵-R¹⁶- représente de préférence un groupe isopropylydène, benzylidène, diphényl méthylidène, cyclohexyl méthylidène, éventuellement substitués, par exemple un groupe 4-méthoxybenzylidène, ou un groupe alkylène linéaire tel que le groupe éthylène.

4. Composé pour son utilisation selon l'une quelconque des revendications précédentes, **caractérisé en ce qu'**il est choisi parmi les composés de formule (2) ou (3) : 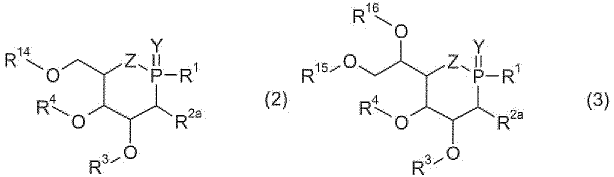 dans lesquelles R¹, R^{2a}, R³, R⁴, Y et Z sont tels que définis dans la revendication 1, R¹⁴, R¹⁵ et R¹⁶ représentent, indépendamment les uns des autres, un atome d'hydrogène, un groupe aryle, hétéroaryle, un groupe alkyle ou cycloalkyle optionnellement substitué, un groupe trichloroacétimidate, un groupe acyle, formyle, sulfonyle, sulfinyle, *tert*-butyldiphénylsilyle, allyle, ester, amide, thioamide, sulfonamide, saccharyle ou bien R¹⁵ et R¹⁶, pris conjointement, forment un radical bivalent de formule -R¹⁵-R¹⁶-.

5. Composé pour son utilisation selon l'une quelconque des revendications précédentes, **caractérisé en ce qu'**il est choisi parmi les composés de formule : 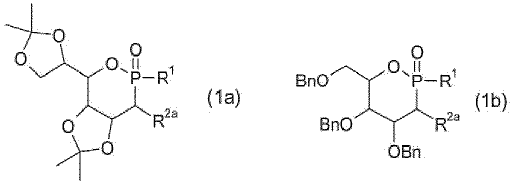 dans laquelle R¹, R^{2a} sont tels que définis dans la revendication 1, et Bn représente le groupe benzyle.

6. Composé pour son utilisation selon l'une quelconque des revendications précédentes, **caractérisé en ce qu'**il est choisi parmi les composés de formule : 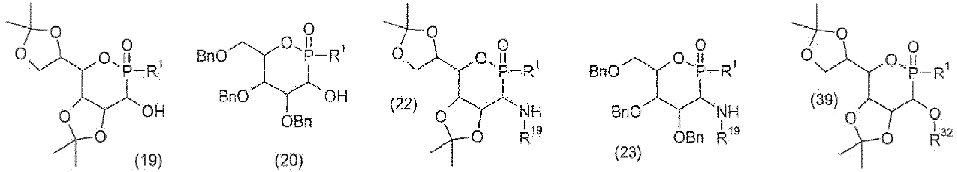 dans laquelle R¹ est tel que défini dans la revendication 1, Bn représente le groupe benzyle, R¹⁹ et R³² représentent indépendamment l'un de l'autre un atome d'hydrogène, un groupe aryle ou hétéroaryle, un groupe alkyle ou cycloalkyle optionnellement substitué, un groupe acyle.

7. Composé pour son utilisation selon l'une quelconque des revendications 1 à 5, **caractérisé en ce que** R^{2a} représente un groupe -X-R², où X = NH.

8. Composé pour son utilisation selon la revendication 7, **caractérisé en ce que** X-R² est NHC(O)R¹², où R¹² représente un groupe aryle ou hétéroaryle, un groupe alkyle ou cycloalkyle optionnellement substitué.

9. Composé pour son utilisation selon l'une quelconque des revendications 1 à 5, **caractérisé en ce que** R^{2a} représente un groupe -X-R², où R² est un groupe aryle, hétéroaryle, de préférence un groupe hétéroaryle.

10. Composé pour son utilisation selon l'une quelconque des revendications 1 à 5, **caractérisé en ce que** R^{2a} représente un atome d'halogène, un groupe carbonate ou un groupe 1H-[1,2,3]triazolyle ou **en ce que** R³ ou R⁴ représente un groupe saccharyle, le groupe saccharyle dérivant de sucres ou de leurs analogues substitués ou déoxy.

11. Composé pour son utilisation selon l'une quelconque des revendications 1 à 3, **caractérisé en ce qu'**il est choisi parmi les composés de formule (27a), (27b), (4.2a) et (5.6d) : 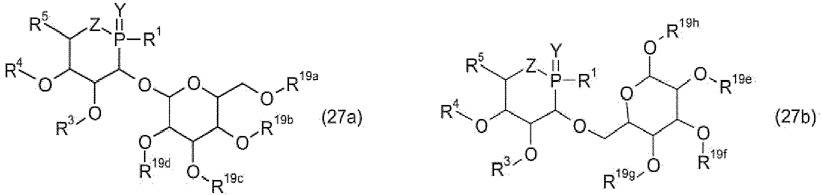 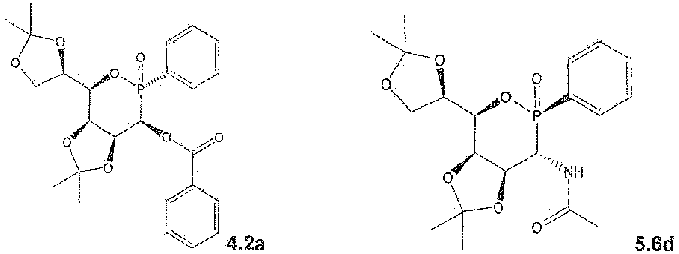 dans lesquelles R^{19a}, R^{19b}, R^{19c}, R^{19d} R^{19e}, R^{19f} et R^{19g} désignent indépendamment les uns des autres un atome d'hydrogène ou le groupe benzyle, R^{19h} désigne un atome d'hydrogène ou le groupe méthyle, les autres substituants étant tels que définis précédemment.

12. Composition pharmaceutique pour une utilisation en vue de réduire ou prévenir l'apparition de métastases chez un patient atteint d'un cancer, **caractérisée en ce qu'**elle comprend au moins un composé de formule (1) tel que défini dans l'une quelconque des revendications précédentes, en association avec un ou plusieurs excipients et/ou véhicules pharmaceutiquement acceptables.

13. Composition pharmaceutique pour son utilisation selon la revendication 12, **caractérisée en ce que** le
patient est atteint d'un cancer choisi parmi le glioblastome multiforme, le cancer du sein et le cancer du poumon non à petites cellules, de préférence le glioblastome multiforme.

14. Composition pharmaceutique pour son utilisation selon l'une quelconque des revendications 12 ou 13,
**caractérisée en ce qu'**elle comprend au moins un solvant organique et au moins un surfactant.

15. Produit de combinaison pour une utilisation dans le traitement d'un cancer, **caractérisé en ce qu'**il comprend :
i) Au moins un composé cytotoxique, et
ii) Au moins un composé de formule (1) tel que défini dans l'une quelconque des revendications 1 à 11 pour une utilisation en vue de réduire ou prévenir l'apparition de métastases.

## Patentansprüche

1. Verbindung zur Verwendung zur Verringerung oder Prävention des Auftretens von Metastasen bei einem an Krebs leidenden Patienten, die der nachstehenden allgemeinen Formel entspricht: 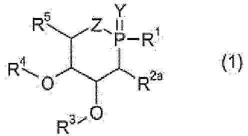 in der:
Y für ein Sauerstoff-, Schwefel- oder Selenatom, vorzugsweise ein Sauerstoffatom, steht,
Z für O, S, Se, NH oder eine Gruppe NR⁶, wobei R⁶ für eine gegebenenfalls substituierte Aryl-, Heteroaryl-, Alkyl- oder Cycloalkylgruppe steht, vorzugsweise ein Sauerstoffatom, steht,
R¹ für ein Wasserstoffatom, eine gegebenenfalls substituierte Alkyl- oder Cycloalkylgruppe oder eine Aryl- oder Heteroarylgruppe steht,
R^{2a} für ein Wasserstoff- oder Halogenatom, eine Azidgruppe (N₃), eine Carbonat- oder Dithiocarbonatgruppe, eine 1H-[1,2,3]Triazolylgruppe oder eine Gruppe -X-R² steht, wobei X für ein Sauerstoff-, Schwefel- oder Selenatom oder eine Gruppe NH oder NR⁷ steht, wobei R⁷ für eine gegebenenfalls substituierte Aryl-, Heteroaryl-, Alkyl- oder Cycloalkylgruppe steht, wobei X vorzugsweise für O oder NH steht und wobei R² für eine gegebenenfalls substituierte Aryl-, Heteroaryl-, Alkyl- oder Cycloalkylgruppe, ein Wasserstoffatom, eine Trichloracetimidatgruppe (-C(=NH)CCl₃) oder eine Acyl-, Förmyl-, Sulfonyl-, Sulfinyl-, *tert*-Butyldiphenylsilyl-, Allyl-, Saccharyl-, Ester-, Amid-, Thioamid- oder Sulfonamidgruppe steht, oder auch X-R² für eine Gruppe P(O)R^{2'}R^{6'} steht, wobei R^{2'} und R^{6'} unabhängig voneinander eine gegebenenfalls substituierte Aryl-, Heteroaryl-, Alkyl- oder Cycloalkylgruppe, OH, Alkoxy oder Aryloxy bedeuten,
R³ und R⁴ unabhängig voneinander für eine gegebenenfalls substituierte Aryl-, Heteroaryl-, Alkyl- oder Cycloalkylgruppe, ein Wasserstoffatom, eine Trichloracetimidat-, Acyl-, Formyl-, Sulfonyl-, Sulfinyl-, *tert*-Butyldiphenylsilyl-, Allyl-, Ester-, Amid-, Thioamid-, Sulfonamid- oder Saccharylgruppe stehen, wobei sich die Saccharylgruppe von Zuckern oder deren substituierten Analogen oder Desoxy-Analogen ableitet, oder auch R³ und R⁴ zusammengenommen einen zweiwertigen Rest der Formel -R³-R⁴- bilden, wobei -R³-R⁴- vorzugsweise für eine gegebenenfalls substituierte Isopropyliden-, Benzyliden-, Diphenylmethyliden- oder Cyclohexylmethylidengruppe, vorzugsweise eine 4-Methoxybenzylidengruppe, oder eine lineare Alkylengruppe, vorzugsweise die Ethylengruppe, steht,
R⁵ für ein Wasserstoffatom oder eine Kohlenwasserstoffgruppe mit einem oder mehreren Heteroatomen, die vorzugsweise aus Sauerstoff, Schwefel und Stickstoff, noch besser Sauerstoff, ausgewählt sind, steht.

2. Verbindung zur Verwendung nach Anspruch 1, **dadurch gekennzeichnet, dass** Y = Z = O.

3. Verbindung zur Verwendung nach Anspruch 1 oder 2, **dadurch gekennzeichnet, dass** die Gruppe R⁵ aus den folgenden Gruppen ausgewählt ist: 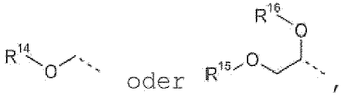 wobei R¹⁴, R¹⁵ und R¹⁶ unabhängig voneinander für ein Wasserstoffatom, eine gegebenenfalls substituierte Aryl-, Heteroaryl-, Alkyl- oder Cycloalkylgruppe, eine Trichloracetimidat-, Acyl-, Formyl-, Sulfonyl-, Sulfinyl-, *tert-*Butyldiphenylsilyl-, Allyl-, Ester-, Amid-, Thioamid-, Sulfonamid- oder Saccharylgruppe stehen, oder auch R¹⁵ und R¹⁶ zusammengenommen einen zweiwertigen Rest der Formel -R¹⁵-R¹⁶- bilden, wobei -R¹⁵-R¹⁶- vorzugsweise für eine gegebenenfalls substituierte Isopropyliden-, Benzyliden-, Diphenylmethyliden- oder Cyclohexylmethylidengruppe, vorzugsweise eine 4-Methoxybenzylidengruppe, oder eine lineare Alkylengruppe, vorzugsweise die Ethylengruppe, steht.

4. Verbindung zur Verwendung nach einem der vorhergehenden Ansprüche, **dadurch gekennzeichnet, dass** sie aus den Verbindungen der Formel (2) oder (3) ausgewählt ist: 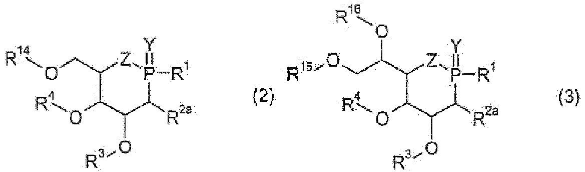 in denen R¹, R^{2a}, R³, R⁴, Y und Z wie in Anspruch 1 definiert sind und R¹⁴, R¹⁵ und R¹⁶ unabhängig voneinander für ein Wasserstoffatom, eine gegebenenfalls substituierte Aryl-, Heteroaryl-, Alkyl- oder Cycloalkylgruppe, eine Trichloracetimidat-, Acyl-, Formyl-, Sulfonyl-, Sulfinyl-, *tert*-Butyldiphenylsilyl-, Allyl-, Ester-, Amid-, Thioamid-, Sulfonamid- oder Saccharylgruppe stehen, oder auch R¹⁵ und R¹⁶ zusammengenommen einen zweiwertigen Rest der Formel -R¹⁵-R¹⁶- bilden.

5. Verbindung zur Verwendung nach einem der vorhergehenden Ansprüche, **dadurch gekennzeichnet, dass** sie aus den Verbindungen der Formel: 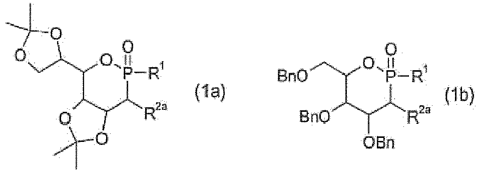 ausgewählt ist, in der R¹ und R^{2a} wie in Anspruch 1 definiert sind und Bn für die Benzylgruppe steht.

6. Verbindung zur Verwendung nach einem der vorhergehenden Ansprüche, **dadurch gekennzeichnet, dass** sie aus den Verbindungen der Formel: 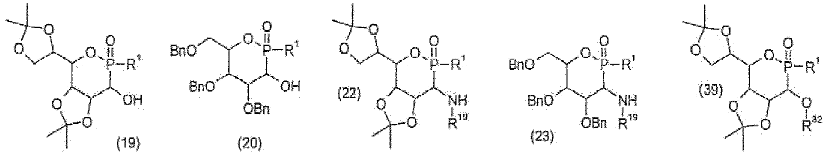 ausgewählt ist, in der R¹ wie in Anspruch 1 definiert ist, Bn für die Benzylgruppe steht und R¹⁹ und R³² unabhängig voneinander für ein Wasserstoffatom, eine Aryl- oder Heteroarylgruppe, eine gegebenenfalls substituierte Alkyl- oder Cycloalkylgruppe oder eine Acylgruppe stehen.

7. Verbindung zur Verwendung nach einem der Ansprüche 1 bis 5, **dadurch gekennzeichnet, dass** R^{2a} für eine Gruppe der Formel -X-R² steht, wobei X = NH.

8. Verbindung zur Verwendung nach Anspruch 7, **dadurch gekennzeichnet, dass** X-R² für NHC(O)R¹² steht, wobei R¹² für eine Aryl- oder Heteroarylgruppe oder eine gegebenenfalls substituierte Alkyl- oder Cycloalkylgruppe steht.

9. Verbindung zur Verwendung nach einem der Ansprüche 1 bis 5, **dadurch gekennzeichnet, dass** R^{2a} für eine Gruppe der Formel -X-R² steht, wobei R² für eine Aryl- oder Heteroarylgruppe, vorzugsweise eine Heteroarylgruppe, steht.

10. Verbindung zur Verwendung nach einem der Ansprüche 1 bis 5, **dadurch gekennzeichnet, dass** R^{2a} für ein Halogenatom, eine Carbonatgruppe oder eine 1H-[1,2,3]Triazolylgruppe steht oder dass R³ oder R⁴ für eine Saccharylgruppe steht, wobei sich die Saccharylgruppe von Zuckern oder deren substituierten Analogen oder Desoxy-Analogen ableitet.

11. Verbindung zur Verwendung nach einem der Ansprüche 1 bis 3, **dadurch gekennzeichnet, dass** sie aus den Verbindungen der Formel (27a), (27b), (4.2a) und (5.6d) : 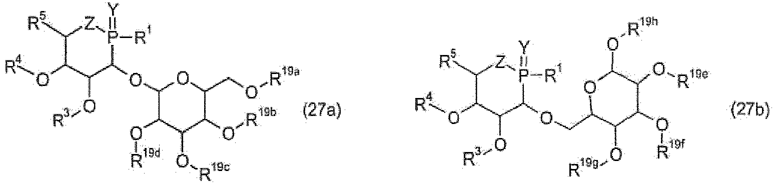 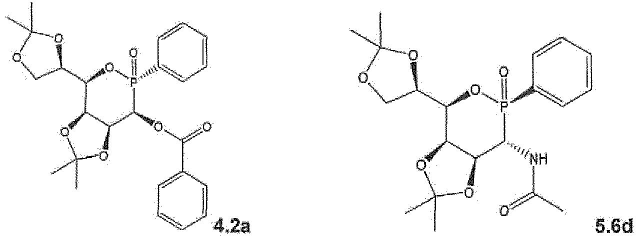 ausgewählt ist, in denen R^{19a}, R^{19b}, R^{19c}, R^{19d}, R^{19e}, R^{19f} und R^{19g} unabhängig voneinander für ein Wasserstoffatom oder die Benzylgruppe stehen und R^{19h} für ein Wasserstoffatom oder die Methylgruppe steht, wobei die anderen Substituenten wie oben definiert sind.

12. Pharmazeutische Zusammensetzung zur Verwendung zur Verringerung oder Prävention des Auftretens von Metastasen bei einem an Krebs leidenden Patienten, **dadurch gekennzeichnet, dass** sie mindestens eine Verbindung der Formel (1) gemäß einem der vorhergehenden Ansprüche in Kombination mit einem oder mehreren pharmazeutisch unbedenklichen Hilfsstoffen und/oder Vehikeln umfasst.

13. Pharmazeutische Zusammensetzung zur Verwendung nach Anspruch 12, **dadurch gekennzeichnet, dass** der Patient an einem Krebs leidet, der aus Glioblastoma multiforme, Brustkrebs und nichtkleinzelligem Lungenkrebs, vorzugsweise Glioblastoma multiforme, ausgewählt ist.

14. Pharmazeutische Zusammensetzung zur Verwendung nach Anspruch 12 oder 13, **dadurch gekennzeichnet, dass** sie mindestens ein organisches Lösungsmittel und mindestens ein Tensid umfasst.

15. Kombinationsprodukt zur Verwendung bei der Behandlung von Krebs, **dadurch gekennzeichnet, dass** sie
i) mindestens eine cytotoxische Verbindung und
ii) mindestens eine Verbindung der Formel (1) gemäß einem der Ansprüche 1 bis 11 zur Verwendung zur Verringerung oder Prävention des Auftretens von Metastasen
umfasst.

## Claims

1. A compound for a use for the purpose of reducing or preventing the appearance of metastases in a patient afflicted with a cancer, corresponding to the general formula below: 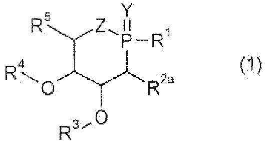 in which:
Y represents an oxygen, sulfur or selenium atom, preferably an oxygen atom,
Z represents O, S, Se, NH or a group NR⁶, in which R⁶ is an optionally substituted aryl or alkyl group, preferably an oxygen atom,
R¹ represents a hydrogen atom, an optionally substituted alkyl or cycloalkyl group, or an aryl or heteroaryl group,
R^{2a} represents a hydrogen or halogen atom, an azide group (N₃), a carbonate or dithiocarbonate group, a 1H-[1,2,3]triazolyl group or a group -X-R², X representing an oxygen, sulfur or selenium atom, a group NH or NR⁷, in which R⁷ is an optionally substituted aryl, heteroaryl, alkyl or cycloalkyl group, X preferably representing O or NH, and R² representing an optionally substituted aryl, heteroaryl, alkyl or cycloalkyl group, a hydrogen atom, a trichloroacetimidate group (-C(=NH)CCl₃), acyl, formyl, sulfonyl, sulfinyl, *tert*-butyldiphenylsilyl, allyl, a saccharyl, ester, amide, thioamide or sulfonamide group, or X-R² represents a group P(O)R^{2'}R^{6'} in which R^{2'} and R^{6'} denote, independently of each other, an optionally substituted aryl, heteroaryl, alkyl or cycloalkyl group, OH, alkoxy or aryloxy,
R³ and R⁴ represent, independently of each other, an optionally substituted aryl, heteroaryl, alkyl or cycloalkyl group, a hydrogen atom, a trichloroacetimidate, acyl, formyl, sulfonyl, sulfinyl, *tert*-butyldiphenylsilyl, allyl, ester, amide, thioamide, sulfonamide or saccharyl group, the saccharyl group deriving from sugars or substituted or deoxy sugar analogues, or alternatively R³ and R⁴, taken together, form a divalent radical of formula -R³-R⁴-, in which -R³-R⁴- preferably represents an isopropylidene, benzylidene, diphenylmethylidene, cyclohexylmethylidene group, optionally substituted, preferably a 4-methoxybenzylidene group, or a linear alkylene group, preferably the ethylene group,
R⁵ represents a hydrogen atom or a hydrocarbon-based group comprising one or more heteroatoms preferably chosen from oxygen, sulfur and nitrogen, better still oxygen.

2. The compound for a use as claimed in claim 1, **characterized in that** Y = Z = O.

3. The compound for a use as claimed in either of claims 1 and 2, **characterized in that** the group R⁵ is chosen from the groups: 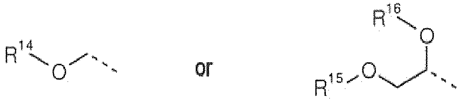 in which R¹⁴, R¹⁵ and R¹⁶ represent, independently of each other, a hydrogen atom, an optionally substituted aryl, heteroaryl, alkyl or cycloalkyl group, a trichloroacetimidate, acyl, formyl, sulfonyl, sulfinyl, *tert*-butyldiphenylsily], allyl, ester, amide, sulfonamide or saccharyl group or alternatively R¹⁵ and R¹⁶, taken together, form a divalent radical of formula -R¹⁵-R¹⁶-, in which -R¹⁵-R¹⁶- preferably represents an isopropylidene, benzylidene, diphenylmethylidene or cyclohexylmethylidene group, which are optionally substituted, for example a 4-methoxybenzylidene group, or a linear alkylene group such as the ethylene group.

4. The compound for a use as claimed in any one of the preceding claims, **characterized in that** it is chosen from the compounds of formulae (2) or (3): 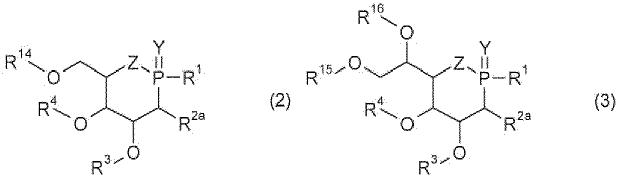 in which R¹, R^{2a}, R³, R⁴, Y and Z are as defined in claim 1, R¹⁴, R¹⁵ and R¹⁶ represent, independently of each other, a hydrogen atom, an aryl or heteroaryl group, an optionally substituted alkyl or cycloalkyl group, a trichloroacetimidate group, an acyl, formyl, sulfonyl, sulfinyl, *tert*-butyldiphenylsilyl, allyl, ester, amide, thioamide, sulfonamide or saccharyl group or alternatively R¹⁵ and R¹⁶, taken together, form a divalent radical of formula -R¹⁵-R¹⁶-.

5. The compound for a use as claimed in any one of the preceding claims, **characterized in that** it is chosen from the compounds of formulae: 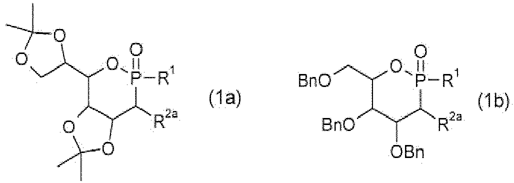 in which R¹ and R^{2a} are as defined in claim 1, and Bn represents the benzyl group.

6. The compound for a use as claimed in any one of the preceding claims, **characterized in that** it is chosen from the compounds of formulae: 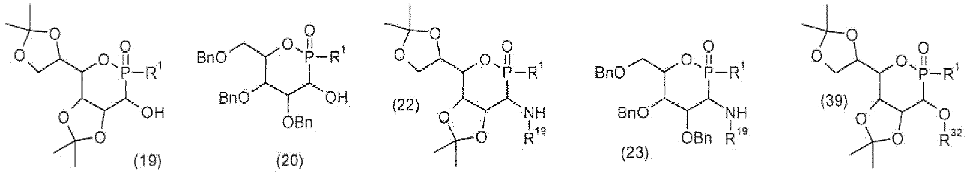 in which R¹ is as defined in claim 1, Bn represents the benzyl group, R¹⁹ and R³² represent, independently of each other, a hydrogen atom, an aryl or heteroaryl group, an optionally substituted alkyl or cycloalkyl group, an acyl group.

7. The compound for a use as claimed in any one of claims 1 to 5, **characterized in that** R^{2a} represents a group -X-R², in which X = NH.

8. The compound for a use as claimed in claim 7, **characterized in that** X-R² is NHC(O)R¹² in which R¹² represents an aryl or heteroaryl group, an optionally substituted alkyl or cycloalkyl group.

9. The compound for a use as claimed in any one of claims 1 to 5, **characterized in that** R^{2a} represents a group -X-R², in which R² is an aryl or heteroaryl group, preferably a heteroaryl group.

10. The compound for a use as claimed in any one of claims 1 to 5, **characterized in that** R^{2a} represents a hydrogen atom, a carbonate group or a 1H-[1,2,3]triazolyl group or R³ or R⁴ represents a saccharyl group, the saccharyl group deriving from sugars or substituted or deoxy sugar analogues.

11. The compound for a use as claimed in any one of claims 1 to 3, **characterized in that** it is chosen from the compounds of formulae (27a), (27b), (4.2a) and (5.6d): 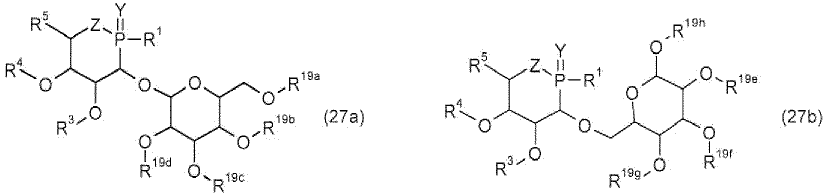 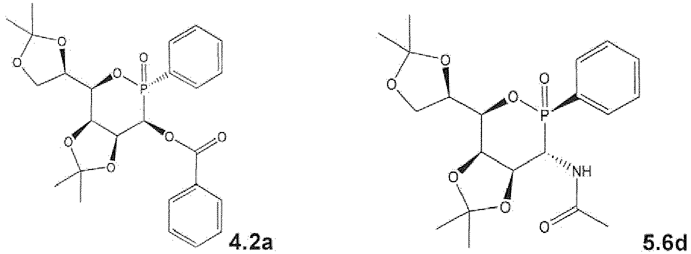 in which R^{19a}, R^{19b}, R^{19c}, R^{19d} R^{19e}, R^{19f} and R^{19g} represent, independently of each other, a hydrogen atom or the benzyl group, R^{19h} represents a hydrogen atom or the methyl group, the other substituents being such as defined previously.

12. A pharmaceutical composition for a use for the purpose of reducing or preventing the appearance of metastases in a patient afflicted with a cancer, **characterized in that** it comprises at least one compound of formula (1) as defined in any one of the preceding claims, in combination with one or more pharmaceutically acceptable excipients and/or vehicles.

13. The pharmaceutical composition for a use according to claim 12, **characterized in that** the patient is afflicted with a cancer chosen from glioblastoma multiforme, breast cancer and non-small-cell lung cancer, preferably glioblastoma multiforme.

14. The pharmaceutical composition for a use as claimed in either of claims 12 and 13, **characterized in that** it comprises at least one organic solvent and at least one surfactant.

15. A combination product for a use in the treatment of a cancer, **characterized in that** it comprises:
i) At least one cytotoxic compound, and
ii) At least one compound of formula (1) as defined in any one of claims 1 to 11, for a use for the purpose of reducing or preventing the appearance of metastases.
